# EUROPÄISCHE PATENTANMELDUNG

(11) **EP 4 183 885 A1**
(43) Veröffentlichungstag der Anmeldung: **24.05.2023**
(21) Anmeldenummer: 22206169.9
(22) Anmeldetag: 08.11.2022
(51) Int. Cl.: C12Q 1/10, C12Q 1/04, G01N 33/68, G16C 20/20, G16B 40/10, G16B 30/10, G16B 20/00, G16B 35/20

(54) **REFERENZDATENSATZ-BASIERTES, SPEKTROMETRISCHES CHARAKTERISIEREN VON ZELLSUBSTRATEN UNTER VERWENDUNG VON TEILBIBLIOTHEKEN**

(30) Priorität: 19.11.2021 DE 102021130356
(71) Anmelder: Bruker Daltonics GmbH & Co. KG, 28359 Bremen (DE)
(72) Erfinder: Sparbier, Katrin, 28357 Bremen (DE)
(74) Vertreter: Boßmeyer, Jens

(57) **Zusammenfassung**

Die Erfindung betrifft Verfahren zum spektrometrischen Charakterisieren eines Test-Zellsubstrats. Die Charakterisierung umfasst die taxonomische Einordnung und die Bestimmung einer interessierenden Eigenschaft des Test-Zellsubstrats. Die Charakterisierung fußt bevorzugt auf massenspektrometrischen Messdaten. Die interessierende Eigenschaft ist bevorzugt eine Resistenz oder Suszeptibilität gegenüber einem wachstumsbeeinflussenden Faktor. Nach Abgleich erster spektrometrischer Messdaten des Test-Zellsubstrats mit einer bereitgestellten Referenzbibliothek wird eine Teilbibliothek erstellt, die solche Referenzdatensätze aus der Referenzbibliothek umfasst, die als eine taxonomische Einordnung des Test-Zellsubstrats erlaubend eingestuft werden. Zweite spektrometrische Messdaten nach einer zweiten Aufbereitung des Test-Zellsubstrats unter Bedingungen, die der Bestimmung einer interessierenden Eigenschaft des Test-Zellsubstrats dienen, werden mit der Teilbibliothek abgeglichen und erlauben eine verlässliche Bestimmung der interessierenden Eigenschaft. In weiteren Ausführungsformen ist die Erstellung weiterer Teilbibliotheken vorgesehen, die als Prozesskontrollen oder zur Bestimmung eines inhomogenen Zellsubstrats verwendet werden können.

## Beschreibung

### Gebiet der Erfindung

Die Erfindung betrifft ein Verfahren zur spektrometrischen Charakterisierung interessierender Eigenschaften von Zellsubstraten unter Erstellung von Messdatensatz-basierten Teilbibliotheken aus einer bereitgestellten Bibliothek, die zur taxonomischen Einordnung des Zellsubstrats geeignete Referenzdatensätze enthält, wobei zur Bestimmung der Eigenschaft des Test-Zellsubstrats ein Abgleich von spektrometrischen Messdaten mit der Bibliothek und mindestens einer daraus erzeugten Teilbibliothek erfolgt.

### Hintergrund der Erfindung

Der Stand der Technik wird im Folgenden mit Bezug auf einen speziellen Aspekt erläutert. Dies soll jedoch nicht als Einschränkung der nachfolgend offenbarten Erfindung verstanden werden. Nützliche Fortentwicklungen und Änderungen vom aus dem Stand der Technik Bekannten können auch über den vergleichsweise engen Rahmen dieser Einleitung hinaus anwendbar sein und werden sich geübten Praktikern auf diesem Gebiet nach der Lektüre der dieser Einleitung nachfolgenden Offenbarung der Erfindung umstandslos erschließen.

Es ist bekannt, Mikroorganismen spektrometrisch zu charakterisieren und taxonomisch einzuordnen. Ein Beispiel ist eine Massen-Flugzeitanalyse (*time-of-flight,* TOF) unter Verwendung von Ionisierung mittels matrix-unterstützter Laserdesorption (*matrix-assisted laser desorption*/*ionisation,* MALDI), siehe den Übersichtsartikel Fenselau und Demirev, Characterization of Intact Microorganisms by Maldi Mass Spectrometry, Mass Spectrometry Reviews, 2001, 20, 157 - 171. Diese Methode liefert für viele Mikroorganismen wie Bakterien und Pilze ohne jegliche Vorkenntnisse im Wesentlichen auf Basis der Massensignatur ribosomaler Proteine verlässliche Ergebnisse auf einer taxonomischen Ebene bis herunter zur Gattung (Genus) oder Art (Spezies). Kommerzielle Systeme werden z.B. von Bruker, MALDI Biotyper^{®}, und bioMerieux, Vitek^{®} MS, angeboten.

Allgemein beruhen die zu Grunde liegenden Charakterisierungsalgorithmen oft auf Vergleichen von aus gemessenen Massenspektren abgeleiteter Information, z.B. Peaklisten, mit Referenzdaten aus einer Bibliothek oder Datenbank und sind im Lauf der Zeit vielfältig weiterentwickelt worden. Die Patentveröffentlichung DE 10 2009 032 649 A1 (entsprechend GB 2 471 746 A und US 2011/0012016 A1) erläutert z.B. ein zweistufiges Identifizierungsverfahren, mit dem Mikroben auf der Ebene der Art oder Unterart identifiziert werden können, auch wenn diese sehr ähnliche Referenzspektren aufweisen. Die Patentveröffentlichung DE 10 2010 006 450 A1 (entspricht US 2011/0202282 A1 und EP 2 354 796 A1) betrifft ebenfalls die Identifizierung von Mikroben in einer Probe durch Berechnungen der Ähnlichkeiten eines Massenspektrums der Probe zu den Referenzspektren in großen Spektrenbibliotheken unter Verwendung eines mehrstufigen Verfahrens.

Die Patentveröffentlichung WO 2017/069935 A1 bezieht sich auf ein Verfahren zur Identifizierung von Mikroorganismen durch MALDI-TOF-Massenspektrometrie, umfassend das Erfassen eines MALDI-Massenspektrums eines Mikroorganismus, das Erfassen von Peaks im erfassten MALDI-Spektrum, das Erzeugen einer Peak-Liste, die Masse und Intensität der erfassten Peaks im Spektrum umfasst, das Erfassen einer Datenbank von Proteinsequenzen, die von DNA-Sequenzen abgeleitet sind, das Erzeugen einer Unterdatenbank von ribosomalen Proteinen aus den Proteinsequenzen und ihren Massen in der Datenbank, Abgleichen der Massen der detektierten Peaks im erfassten MALDI-Spektrum mit den Massen der ribosomalen Proteine in der erzeugten Unterdatenbank, Bewerten der oben erhaltenen Übereinstimmungen für jeden dargestellten Mikroorganismus, Erzeugen einer Peak-Liste der genauen Massen der übereinstimmenden ribosomalen Proteine, Rekalibrieren der Peak-Liste, die Masse und Intensität umfasst, mit der Peak-Liste der genauen Massen der übereinstimmenden ribosomalen Proteine, Identifizieren eines Mikroorganismus mit der höchsten Punktzahl und Wiederholen, bis eine gewünschte Verbesserung in der rekalibrierten Peak-Liste oder eine validierte Identifizierung erreicht ist.

Ein weiteres Beispiel für spektrometrische Charakterisierung von Mikroorganismen ist eine absorptionsspektrometrische Analyse unter Verwendung infraroten Lichts, siehe z.B. die Monografie von Dieter Naumann: Infrared Spectroscopy in Microbiology, Encyclopedia of Analytical Chemistry, pp. 102-131, 2000. Diese Methode hat sich insbesondere für Subtypisierung unterhalb der taxonomischen Ebene der Art bewährt, bei entsprechender Vorkenntnis der Art des Mikroorganismus, die beispielweise mittels massenspektrometrischer Analyse erhalten werden kann (DE 10 2013 022 016 A1, entsprechend WO 2015/090727 A1). Ein Beispiel für ein kommerzielles System auf diesem Gebiet ist IR Biotyper^{®} von Bruker.

Auch hier hat es Weiterentwicklungen gegeben. Die Patentveröffentlichung DE 11 2005 001 530 T5 (entsprechend WO 2006/002537 A1) zum Beispiel beschreibt ein Verfahren für die Identifikation von Mikroorganismen, das das Erhalten mindestens eines Spektralbildes des Mikroorganismus mit mehreren Pixeln und das Auswählen von einem oder mehreren Spektren aus dem Spektralbild mit mehreren Pixeln auf der Basis von vorbestimmten Spektraleigenschaften umfasst, wobei die ausgewählten Spektren Spektralinformationseigenschaften des Mikroorganismus umfassen.

Darüber hinaus ist es bekannt, weitere Eigenschaften eines Mikroorganismus spektrometrisch zu charakterisieren, insbesondere die Empfindlichkeit oder Widerstandskraft gegenüber einer bioaktiven Substanz, z.B. einem antimikrobiellen Mittel wie einem Antibiotikum oder Antimykotikum. Phänotypische oder zellbezogene Ansätze beruhen darauf, Mikroorganismenwachstum oder dessen Ausbleiben in Gegenwart einer bioaktiven Substanz nachzuweisen, wobei die verlässliche taxonomische Zuordnung der Art eine wichtige Rolle spielt (DE 10 2006 021 493 A1, entsprechend GB 2 438 066 A und US 2008/0009029 A1).

Andere Ansätze können dagegen substanzbezogen sein und etwaige durch Co-Inkubation des Mikroorganismus hervorgerufene Änderungen der bioaktiven Substanz oder des umgebenden Inkubationsmilieus nachweisen. Ein Beispiel ist die Hydrolyse eines β-Lactam-Antibiotikums durch einen β-Lactamase ausscheidenden Mikroorganismus (DE 10 2010 023 452 A1, entsprechend WO 2011/154517 A1) oder der Verstoffwechslungsgrad eines Nährstoffs, der isotopisch markiert sein kann, durch den Mikroorganismus (EP 2 801 825 A1, entsprechend US 2014/0335556 A1 oder DE 10 2014 000 646 A1, entsprechend WO 2015/107054 A1).

Ein weiterer Ansatz ist in Patentveröffentlichung EP 2 806 275 A1 (entspricht WO 2014/187517 A1) offenbart. Ein massenspektrometrisches Verfahren zur Bestimmung der mikrobiellen Resistenz gegen Antibiotika beinhaltet die Kultivierung von Mikroben in einem Antibiotikum-versetzten Medium, das ebenfalls eine dosiert zugegebene Referenzsubstanz enthält, und die Aufnahme von Massenspektren der Mikroben einschließlich Referenzsubstanz nach der Kultivierung sowie die anschließende Bewertung des Wachstums an Hand der Signale der Referenzsubstanz in den Massenspektren.

Die Patentveröffentlichung WO 2018/099500 A1 erläutert Verfahren zur Aufbereitung lebendiger, mikrobieller Proben und Mikroorganismen für eine anschließende massenspektrometrische Messung und Auswertung. Die Aufbereitung kann direkt auf einem massenspektrometrischen Probenträger stattfinden.

Weitere beachtenswerte Veröffentlichungen umfassen: E.A. Idelevich et al., Rapid detection ofantibiotic resistance by MALDI-TOF mass spectrometry using a novel direct-on-target microdroplet growth assay, Clinical Microbiology and Infection 24 (2018) 738-743; M. Li et al., Rapid antimicrobial susceptibility testing by matrix-assisted laser desorption ionization-time of flight mass spectrometry using a qualitative method in Acinetobacter baumannii complex, Journal of Microbiological Methods 153 (2018) 60-65; Idelevich et al., Rapid Direct Susceptibility Testing from Positive Blood Cultures by the Matrix-Assisted Laser Desorption Ionization-Time of Flight Mass Spectrometry-Based Direct-on-Target Microdroplet Growth Assay, Journal of Clinical Microbiology, October 2018 Volume 56 Issue 10 e00913-18; Ioannis K. Neonakis et al., Detection of carbapenemase producers by matrix-assisted laser desorption-ionization time-of-flight mass spectrometry (MALDI-TOFMS), European Journal of Clinical Microbiology & Infectious Diseases 2019, https://doi.org/10.1007/s10096-019-03620-0; Nix et al., Methicillin Resistance Detection by MALDI-TOF MS, Frontiers in Microbiology, 1 February 2020, Volume 11, Article 232; und Timothy S. Horseman et al., Rapid qualitative antibiotic resistance characterization using VITEK MS, Diagnostic Microbiology and Infectious Disease Volume 97, Issue 4, August 2020, 115093.

Es besteht daher ein Bedarf, ein verbessertes Verfahren für eine spektrometrische Charakterisierung von Zellsubstraten von Interesse und insbesondere Mikroorganismen von Interesse bereitzustellen. Weitere von der Erfindung zu lösende Aufgaben ergeben sich für den Fachmann ohne weiteres bei der Lektüre der nachfolgenden Offenbarung.

### Zusammenfassung der Erfindung

Die Erfindung betrifft Verfahren zum spektrometrischen Charakterisieren eines Test-Zellsubstrats, bevorzugt eines Test-Mikroorganismus. Die Charakterisierung fußt bevorzugt auf massenspektrometrischen Messdaten. Die Erfindung beruht auf der Erkenntnis, dass aus einer bereitgestellten Bibliothek, die Referenzdatensätze enthält, die eine taxonomische Einordnung des Test-Zellsubstrats ermöglichen, nach einer ersten erfolgreichen taxonomischen Einordnung eines zu testenden Zellsubstrats, d.h. nach einem ersten Abgleich der Messdaten mit den Referenzdatensätzen in der Bibliothek und hinreichender Ähnlichkeit zwischen den Messdaten und mindestens einem Referenzdatensatz aus der Bibliothek, wodurch eine taxonomische Einordnung ermöglicht wird, Teilbibliotheken erstellt werden können, mit denen weitere spektrometrische Messdaten des gleichen Zellsubstrats aus wenigstens einer weiteren experimentellen Aufbereitung abgeglichen werden können. Insbesondere können solche Teilbibliotheken verwendet werden, die solche spektrometrische Referenzdatensätze umfassen, die als in einem ersten Abgleichergebnis der spektrometrischen Messdaten des Test-Zellsubstrats eine taxonomische Einordnung des Test-Zellsubstrats erlaubend bewertet worden sind. Durch die Verwendung solcher Teilbibliotheken für die spektrometrische Charakterisierung eines Test-Zellsubstrats wird die Spezifität der taxonomischen Einordnung des Test-Zellsubstrats in den weiteren Aufbereitungen und die Bestimmung von Eigenschaften des Test-Zellsubstrats, die mit Hilfe der experimentellen Aufbereitungen bestimmt werden sollen, verbessert, da die zuverlässige taxonomische Einordnung eines Test-Zellsubstrats für die Eigenschaftsbestimmung auf Grundlage spektrometrischer Messdaten eine wichtige Rolle spielt.

Das erste Abgleichergebnis ermöglicht es beispielsweise, aus der Bibliothek solche Teilbibliotheken bereitzustellen, die solche Referenzdatensätze umfassen, die dem spektrometrischen Messergebnis des Zellsubstrats ähnlich sind, d.h. die einen hinreichend hohen Übereinstimmungsgrad aufweisen, so dass eine taxonomische Einordnung des Zellsubstrats durch Abgleich möglich ist, oder die dem spektrometrischen Messergebnis unähnlich sind, d.h. nicht hinreichend Übereinstimmung mit den spektrometrischen Messdaten aufweisen, um eine taxonomische Einordnung, insbesondere der Art oder Gattung, zu ermöglichen.

Eine erste Teilbibliothek, die solche Referenzdatensätze aus der Bibliothek mit einem hohen Übereinstimmungsgrad zu den spektrometrischen Messdaten umfasst, die als eine taxonomische Einordnung erlaubend bewertet werden, kann zur taxonomischen Einordnung und zur Bestimmung von Eigenschaften des gleichen Test-Zellsubstrats verwendet werden, wenn das Test-Zellsubstrat unter Bedingungen aufbereitet wird, die sich von denen der ersten Aufbereitung unterscheiden. Dabei werden die spektrometrischen Messdaten des Test-Zellsubstrats aus der weiteren Aufbereitung in wenigstens einem weiteren Abgleichergebnis mit den Referenzdatensätzen aus der ersten Teilbibliothek verglichen. Dadurch wird die Spezifität der taxonomischen Einordnung aufgrund dieses weiteren Abgleichergebnisses verbessert, was sich wiederrum positiv auf die spektrometrische Bestimmung einer Eigenschaft des Test-Zellsubstrats auswirkt, die sich durch die im Vergleich zur ersten Aufbereitung nicht deckungsgleichen Bedingungen der weiteren Aufbereitung bestimmt.

Weiterhin wurde erkannt, dass nach einer ersten erfolgreichen taxonomischen Einordnung, neben einer ersten Teilbibliothek weitere Teilbibliotheken aus der bereitgestellten Bibliothek erstellt und zur Verbesserung der Ergebnisse der taxonomischen Einordnung des Test-Zellsubstrats und/oder der Bestimmung von Eigenschaften des Test-Zellsubstrats mit Hilfe weiterer Aufbereitungen verwendet werden können. Ein Beispiel ist eine zweite Teilbibliothek, die nur solche Referenzdatensätze umfasst, die als keine taxonomische Einordnung insbesondere der Art oder der Gattung des Zellsubstrats basierend auf den ersten spektrometrischen Messdaten erlaubend bewertet werden. Diese zweite Teilbibliothek kann als Negativkontrolle verwendet werden, denn ein als eine taxonomische Einordnung (z.B. der Gattung oder Art) erlaubend bewertetes Abgleichergebnis der spektrometrischen Messergebnisse des Test-Zellsubstrats aus einer weiteren Aufbereitung mit den spektrometrischen Referenzdatensätzen der zweiten Teilbibliothek ist nicht zu erwarten, sollte sogar ausgeschlossen sein. Solche Referenzdatensätze, die im ersten Abgleichergebnis als unähnlich bzw. eine taxonomische Einordnung insbesondere der Art oder Gattung nicht-erlaubend bewertet werden, können demzufolge ein wichtige Rolle bei der Analyse und Bewertung der spektrometrischen Messdaten aus weiteren Aufbereitungen spielen, da eine taxonomische Einordnung oder Bestimmung von Eigenschaften für eine vom ersten Abgleichergebnis abweichende taxonomische Einordnung des Test-Zellsubstrats ein Hinweis auf eine Verunreinigung oder Kontamination der Probe oder das Vorhandensein einer Inhomogenität im Test-Zellsubstrat selbst sein können.

Durch das Bereitstellen oder Erstellen von Referenzdatensätzen, die spezifisch für ein Zellsubstrat und abhängig von einem ersten Abgleichergebnis der spektrometrischen Messdaten mit einer umfassenden, allgemeinen Bibliothek ausgewählt werden, in mindestens einer Teilbibliothek, die zum Abgleich bei weiteren spektrometrischen Analysen des gleichen Test-Zellsubstrats verwendet wird, kann die Spezifität der taxonomischen Einordnung des unter veränderten Bedingungen aufbereiteten (z.B. kultivierten) Test-Zellsubstrats und die Bestimmung von Eigenschaften des Test-Zellsubstrats weiter verbessert werden.

Das Verfahren umfasst: Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen. Die Bibliothek umfasst insbesondere Referenzdatensätze, die für ein verwendetes spektrometrisches Nachweisverfahren spezifisch sind und sich von Referenzdatensätzen anderer spektrometrischer Nachweisverfahren unterscheiden, so dass sie untereinander nicht kompatibel sind. Referenzdatensätze können spektrometrische Messdaten oder davon abgeleitete Daten umfassen, die von bekannten Zellsubstraten und insbesondere Mikroorganismen erhalten wurden. Eine bevorzugte Bibliothek umfasst Referenzdatensätze spektrometrischer Nachweisverfahren von Mikroorganismen, weiter bevorzugt Referenzdatensätze massenspektrometrischer Nachweisverfahren von *Enterobacteriaceae,* am meisten bevorzugt Referenzdatensätze massenspektrometrischer Nachweisverfahren von *Enterobacteriaceae,* die mit einem validierten MALDI-Flugzeit-System erstellt wurden, z.B. mit dem MALDI Biotyper^{®} (Bruker).

Ein Referenzdatensatz kann ein Spektrum oder ein aus einem Spektrum abgeleitetes Daten-n-tupel umfassen. Ein Beispiel für ein Daten-n-tupel ist eine Liste aus Häufigkeitsinformation (Abundanz) im Spektralsignal und dieser zugeordneter enger Massenkanäle, gegebenenfalls zuzüglich etwaiger Metainformation zum Spektrum. Ein weiteres Beispiel für ein Daten-n-tupel ist eine Liste aus Absorptionsinformation im Spektralsignal und dieser zugeordneter enger Wellenzahlkanäle, gegebenenfalls zuzüglich etwaiger Metainformation zum Spektrum. Abgeleitete Daten können beispielsweise aus den ursprünglichen Spektren erzeugte Peaklisten der prominentesten Spektralsignale oder anderweitig reduzierte Daten enthalten, z.B. mittels Grundliniensubtraktion, Ableitung, Rauschentfernung und dergleichen.

Das Verfahren umfasst ferner: Bereitstellen oder Erstellen erster spektrometrischer Messdaten vom Test-Zellsubstrat nach einer ersten Aufbereitung. Eine Aufbereitung ist ein Arbeitsablauf, mit dem die anfangs verfügbare Biomasse des zu untersuchenden Test-Zellsubstrats verarbeitet und für eine spektrometrische Messung vorbereitet wird. Das Bereitstellen oder Erstellen erster spektrometrischer Messdaten kann eine wiederholte spektrometrische Messung des Test-Zellsubstrats nach einer ersten Aufbereitung umfassen, dabei kann die Messung 1-, 2-, 3-, 4- oder 5-mal, bevorzugt 3-mal, wiederholt werden. Für das Bereitstellen oder Erstellen der ersten spektrometrischen Messdaten wird dann typischerweise der Median der Messungen oder ein anderer geeigneter statistischer Lageparameter bestimmt und verwendet. Diese Wiederholungen können unter Verwendung biologischer Replikate des Test-Zellsubstrats und/oder technischer Replikate aus derselben Aufbereitung entstanden sein. Die erste Aufbereitung kann einen Vermehrungsschritt des Test-Zellsubstrats enthalten, um die verfügbare Biomasse zu vergrößern und damit die nachweisbaren Spektralsignale gegenüber allgegenwärtigem Hintergrund oder Rauschen in spektrometrischen Messdaten stärker hervorzuheben. Das Bereitstellen oder Erstellen spektrometrischer Messdaten kann eine massenspektrometrische Analyse aufweisen, beispielsweise unter Verwendung einer desorbierenden Ionisierung von Biomasse des Test-Zellsubstrats nach erfolgter Aufbereitung, weiter gefolgt von einer massendispergierenden Analyse der erzeugten Ionen, insbesondere eine MALDI-Flugzeitanalyse (MALDI-TOF). Die erste Aufbereitung kann unmittelbar auf einem Probenträger erfolgen, der als Substrat für das Bereitstellen oder Erstellen spektrometrischer Messdaten dient, z.B. einem MALDI-Probenträger wie AnchorChip^{™} (Bruker) oder MBT Biotarget^{™} (Bruker) für massenspektrometrische Messungen oder einem Objektträger aus Glas oder Keramik.

Das Verfahren umfasst weiterhin: Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Test-Zellsubstrats oder davon abgeleiteten Daten aufgeführt sind. Beispiele für Abgleichergebnisse sind Listen mit Ähnlichkeitsmaßzahlen ("scores") wie sie von kommerziellen Systemen wie MALDI Biotyper^{®} (Bruker) verwendet werden. Der Logarithmus dieser Ähnlichkeitsmaßzahl ("log(score)") fällt in die Bereiche (i) größer als oder gleich 2.0, was laut Anbieter als zuverlässige Bestimmung der Art des untersuchten Test-Zellsubstrats bewertet wird, (ii) kleiner als 2.0 und größer als oder gleich 1.7, was als zuverlässige Bestimmung der Gattung des untersuchten Test-Zellsubstrats bewertet wird, und (iii) kleiner als 1.7, was als fehlgeschlagene Bestimmung bewertet wird. Mit dem MALDI Biotyper^{®} System können auch taxonomische Einordnungen auf anderen taxonomischen Ebenen wie der Unterart (Subspezies) vorgenommen werden. Für eine zuverlässige Bestimmung der Unterart kann der Bereich (i) weiter differenziert werden. Andere Anbieter verwenden Klassifikationen ähnlicher Art.

Das Verfahren umfasst außerdem: Bereitstellen oder Erstellen einer Teilbibliothek beinhaltend solche Referenzdatensätze aus der Bibliothek, für die das erste Abgleichergebnis als eine taxonomische Einordnung des Test-Zellsubstrats erlaubend bewertet wird. Die taxonomische Einordnung kann eine Zuordnung eines Taxons zum Test-Zellsubstrat umfassen, das aus der Gruppe gewählt ist: Gattung (Genus), Art (Spezies), Unterart (Subspezies) und Varietät oder Serotyp. Bevorzugt umfasst die taxonomische Einordnung eine Zuordnung eines Taxons zum Test-Zellsubtrat, das aus der Gruppe Gattung (Genus) und/oder Art (Spezies) gewählt ist. Die taxonomische Einordnung einer Unterart (Subspezies), einer Varietät oder eines Serotyps kann von Interesse sein, wenn die Varietät oder der Serotyp eine andere Eigenschaft von Interesse aufweist, wie z.B. eine andere Pathogenität oder Resistenz/Suszeptibilität. Es kann vorgesehen sein, dass eine Teilbibliothek nur denjenigen Referenzdatensatz aus der Bibliothek enthält, der im ersten Abgleichergebnis die höchste Ähnlichkeitsmaßzahl aufweist, d.h. denjenigen Referenzdatensatz, der den höchsten Übereinstimmungsgrad mit den spektrometrischen Messdaten oder davon abgeleiteten Daten der ersten Aufbereitung des Zellsubstrats aufweist (und als eine taxonomische Einordnung erlaubend bewertet wird). Es kann vorgesehen sein, dass eine Teilbibliothek die 1, 2, 3, 4, 5, 6, 7, 8, 9 oder 10, bevorzugt die 1, 2, 3, 4 oder 5, besonders bevorzugt die 1, 2 oder 3 Referenzdatensätze umfasst, am meisten bevorzugt die 3 Referenzdatensätze umfasst, die im ersten Abgleichergebnis die höchsten Übereinstimmungsgrade aufgewiesen haben (und als eine taxonomische Einordnung erlaubend bewertet werden). Es kann vorgesehen sein, diejenigen Referenzdatensätze, für die das erste Abgleichergebnis als keine taxonomische Einordnung des Test-Zellsubstrats erlaubend bewertet wird, beim Bereitstellen oder Erstellen einer Teilbibliothek auszuschließen.

Es kann vorgesehen sein, dass nach dem Abgleich der ersten spektrometrischen Messdaten mit der Bibliothek und vor dem Bereitstellen oder Erstellen einer Teilbibliothek, der notwendige Übereinstimmungsgrad in Form der Ähnlichkeitsmaßzahl und/oder des Logarithmus der Ähnlichkeitsmaßzahl, zwischen spektrometrischen Messdaten und Referenzdatensätzen, um als eine taxonomische Einordnung erlaubend bewertet zu werden, angepasst wird. Es kann vorgesehen sein, dass der notwendige Übereinstimmungsgrad zwischen spektrometrischen Messdaten und der Bibliothek, um als eine taxonomische Einordnung erlaubend bewertet zu werden, angepasst wird und/oder der notwendige Übereinstimmungsgrad zwischen spektrometrischen Messdaten und einer Teilbibliothek, um als eine taxonomische Einordnung erlaubend bewertet zu werden, angepasst wird. Eine Erhöhung des notwendigen Übereinstimmungsgrads kann z.B. vorgesehen sein, wenn das erste Abgleichergebnis als eine taxonomische Einordnung in verschiedene Gattungen, z.B. *Citrobacter sp.* und *Escherichia sp.* erlaubend bewertet wird. Durch die Anpassung des notwendigen Übereinstimmungsgrads kann in einem solchen Fall die Zuverlässigkeit einer taxonomischen Einordnung basierend auf dem ersten Abgleichergebnis verbessert werden. Eine Reduktion des notwendigen Übereinstimmungsgrads kann z.B. vorgesehen sein, wenn das erste Abgleichergebnis keine Ähnlichkeitsmaßzahlen enthält, die als eine taxonomische Einordnung, wie eine taxonomische Einordnung der Art, erlaubend bewertet werden, jedoch solche, die als keine taxonomische Einordnung der Art oder der Gattung erlaubend bewertet werden. In diesen Fällen können, z.B. bei Verwendung des MALDI Biotyper^{®} (Bruker), die Bewertungs-Bereiche, in die die Logarithmen der Ähnlichkeitsmaßzahlen hineinfallen und aufgrund derer eine Bewertung als taxonomische Einordnung erlaubende und keine taxonomische Einordnung erlaubende Abgleichergebnisse erfolgt, angepasst werden.

Im Falle des MALDI Biotyper^{®} Systems kann eine Anpassung für die Bereiche (i), (ii) und (iii) zusammen vorgenommen werden. Es kann aber auch nur der Bereich (i), nur die Bereiche (i) und (ii) oder die Bereiche (ii) und (iii) angepasst werden. Der Bereich (i) kann angepasst werden auf einen Wert größer oder gleich 2.5, 2.4, 2.3, 2.2, 2.1, 2.0, 1.9, 1.8, 1.7, 1.6, 1.5, 1.4, 1.3, 1.2, 1.1 oder 1.0, um laut Anbieter eine zuverlässige Bestimmung der Art zu ermöglichen. Ebenso kann der Bereich (ii) angepasst werden auf einen Wert kleiner als 2.5, 2.4, 2.3, 2.2, 2.1, 2.0, 1.9, 1.8, 1.7, 1.6, 1.5, 1.4, 1.3, 1.2, 1.1 oder 1.0 und größer oder gleich 0.7, 0.8, 0.9, 1.0, 1.1, 1.2, 1.3, 1.4, 1.5, 1.6, 1.7, 1.8, 1.9, 2.0, 2.1, oder 2.2, um laut Anbieter eine zuverlässige Bestimmung der Gattung zu ermöglichen. Ebenso kann der Bereich (iii) angepasst werden auf einen Wert kleiner als 0.7, 0.8, 0.9, 1.0, 1.1, 1.2, 1.3, 1.4, 1.5, 1.6, 1.7, 1.8, 1.9, 2.0, 2.1, oder 2.2, um laut Anbieter als eine fehlgeschlagene taxonomische Einordnung, d.h. keine taxonomische Einordnung der Art oder Gattung erlaubend, bewertet zu werden. Die Werte, die für eine Anpassung des Bereichs (i) vorgesehen sind, sind größer als die Werte für den Bereich (ii). Die Werte, die für eine Anpassung des Bereichs (ii) vorgesehen sind, sind kleiner als die Werte für den Bereich (i) und größer als die Werte für den Bereich (iii). Die Werte, die für eine Anpassung des Bereichs (iii) vorgesehen sind, sind kleiner als die Werte für den Bereich (ii) und kleiner als die Werte für den Bereich (i).

In einer Ausführungsform kann vorgesehen sein, dass das Test-Zellsubstrat ein Mikroorganismus ist. Vorzugsweise ist der Mikroorganismus ein Bakterium und insbesondere stammt das Bakterium aus der Familie der *Enterobacteriaceae.* In dieser Ausführungsform kann vorgesehen sein, dass die erste Aufbereitung eine Wachstumskontrolle ist und dass für eine zweite Aufbereitung des Test-Mikroorganismus ein antimikrobielles Mittel wie ein Antimykotikum oder ein Antibiotikum, insbesondere Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN), als wachstumsbeeinflussender Faktor ausgewählt wird.

In einer bevorzugten Ausführungsform stammt das Test-Zellsubstrat aus der Familie der *Enterobacteriaceae* und es ist vorgesehen, dass die erste Aufbereitung eine Wachstumskontrolle ist, und dass für eine zweite Aufbereitung des Test-Mikroorganismus ein antimikrobielles Mittel wie ein Antimykotikum oder ein Antibiotikum, insbesondere Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN), bestimmter Konzentration als wachstumsbeeinflussender Faktor ausgewählt wird. In dieser Ausführungsform können die Referenzdatensätze der Bibliothek und die massenspektrometrischen Messdaten der Aufbereitungen bevorzugt auf Messdaten, die mit dem MALDI Biotyper^{®} (Bruker) oder einem anderen marktgängigen System erstellt wurden, basieren.

In einer Ausführungsform kann vorgesehen sein, dass die bereitgestellte oder erstellte Teilbibliothek beinhaltend Referenzdatensätze aus der Bibliothek, für die das erste Abgleichergebnis als eine taxonomische Einordnung des Test-Zellsubstrats erlaubend bewertet wird, eine erste bereitgestellte oder erstellte Teilbibliothek ist. Neben der ersten Teilbibliothek kann die Bereitstellung oder Erstellung einer zweiten Teilbibliothek vorgesehen sein, wobei eine erste und eine zweite Teilbibliothek nicht deckungsgleich sind. Nicht deckungsgleich heißt insbesondere, dass eine erste Teilbibliothek und eine zweite Teilbibliothek in wenigstens einem Aspekt, wie der Anzahl der enthaltenen Referenzdatensätze und/oder der Identität der Referenzdatensätze, nicht übereinstimmen, wohingegen andere Aspekte durchaus identisch sein können. Eine zweite Teilbibliothek kann diejenigen Referenzdatensätze aus der Bibliothek, für die das erste Abgleichergebnis als keine taxonomische Einordnung insbesondere der Art oder der Gattung des Test-Zellsubstrats erlaubend bewertet wird, umfassen. Eine zweite Teilbibliothek kann aus denjenigen Referenzdatensätzen aus der Bibliothek, für die das erste Abgleichergebnis als keine taxonomische Einordnung (z.B. der Art oder der Gattung) des Test-Zellsubstrats erlaubend bewertet wird, bestehen.

Eine Teilbibliothek enthält Referenzdatensätze, die auch in der Bibliothek enthalten sind. Eine Teilbibliothek enthält insbesondere eine Untermenge der Referenzdatensätze aus der Bibliothek. Eine Teilbibliothek kann eine geringere Anzahl an Referenzdatensätzen enthalten als die Bibliothek. Es ist möglich, dass die Anzahl der Referenzdatensätze in der ersten Teilbibliothek weniger als 10%, bevorzugt weniger als 5%, weiter bevorzugt weniger als 3%, noch weiter bevorzugt weniger als 1% und insbesondere weniger als 5%o, weiter insbesondere weniger als 3%o und noch weiter insbesondere weniger als 1‰ der Anzahl der Referenzdatensätze in der Bibliothek umfasst. Beinhaltet die Bibliothek zum Beispiel 3000 Referenzdatensätze, kann die maximale Anzahl der Referenzdatensätze in der Teilbibliothek in einem Beispiel auf 3 beschränkt werden. Eine erste und eine zweite Teilbibliothek können zusammen dieselbe Anzahl an Referenzdatensätzen enthalten wie die Bibliothek. Eine erste und eine zweite Teilbibliothek können zusammen eine geringere Anzahl an Referenzdatensätzen enthalten als die Bibliothek. Eine erste Teilbibliothek kann dieselbe Anzahl an Referenzdatensätzen enthalten wie eine zweite Teilbibliothek. Eine erste und eine zweite Teilbibliothek können eine Schnittmenge an Referenzdatensätzen aufweisen, d.h. Referenzdatensätze können sowohl in einer ersten als auch einer zweiten Teilbibliothek enthalten sein. Eine zweite Teilbibliothek kann dieselbe, eine höhere oder eine geringere Anzahl an Referenzdatensätzen enthalten als eine erste Teilbibliothek. In einer Ausführungsform kann das Bereitstellen oder Erstellen einer ersten Teilbibliothek vorgesehen sein, die einen Referenzdatensatz der Bibliothek beinhaltet, insbesondere denjenigen Referenzdatensatz aus der Bibliothek beinhaltet, für den das erste Abgleichergebnis als eine taxonomische Einordnung des Test-Zellsubstrats erlaubend bewertet wird und dessen Ähnlichkeitsmaßzahl bzw. Logarithmus der Ähnlichkeitsmaßzahl den höchsten Wert im ersten Abgleichergebnis aufweist (beste Übereinstimmung oder *best match*).

In anderen Ausführungsformen kann die erste Teilbibliothek drei oder fünf Referenzdatensätze umfassen, wobei Referenzdatensätze aus der Bibliothek umfasst sind, die als eine taxonomische Einordnung erlaubend bewertet werden und deren Ähnlichkeitsmaßzahlen bzw. Logarithmen der Ähnlichkeitsmaßzahlen die drei oder fünf höchsten Werte bzw. den höchsten Übereinstimmungsgrad im ersten Abgleichergebnis aufweisen. Die Bibliothek wie die daraus entstandenen Teilbibliotheken sind insbesondere einheitlich mit Referenzdatensätzen bestückt, die für ein bestimmtes spektrometrisches, insbesondere ein massenspektrometrisches, Nachweisverfahren spezifisch sind. Die Bibliothek wie die daraus entstandenen Teilbibliotheken können insbesondere dadurch einheitlich mit Referenzdatensätzen bestückt sein, dass die Referenzdatensätze unter Verwendung spektrometrischer, insbesondere massenspektrometrischer Nachweisverfahren gewonnen oder durch Ableitung aus spektrometrischen oder massenspektrometrischen Messdaten erhalten wurden.

Das Verfahren umfasst ebenso: Bereitstellen oder Erstellen zweiter spektrometrischer Messdaten vom Test-Zellsubstrat nach wenigstens einer zweiten Aufbereitung unter Bedingungen, die mit denen der ersten Aufbereitung nicht deckungsgleich sind. Nicht deckungsgleich heißt insbesondere, dass die Bedingungen der ersten Aufbereitung und zweiten Aufbereitung in wenigstens einem Aspekt nicht übereinstimmen, wohingegen andere Aspekte durchaus identisch sein können. Das Bereitstellen oder Erstellen zweiter spektrometrischer Messdaten kann eine wiederholte spektrometrische Messung des Test-Zellsubstrats nach einer ersten Aufbereitung umfassen, dabei kann die Messung 1-, 2-, 3-, 4- oder 5-mal, bevorzugt 3-mal, wiederholt werden. Für das Bereitstellen zweiter spektrometrischer Messdaten wird dann typischerweise der Median der Messungen oder ein anderer geeigneter statistischer Lageparameter bestimmt und verwendet. Diese Wiederholungen können unter Verwendung biologischer Replikate und/oder technischer Replikate aus derselben Aufbereitung des Test-Zellsubstrats entstanden sein.

Es kann vorgesehen sein, dass die erste Aufbereitung und die zweite Aufbereitung zeitgleich bereitgestellt oder erstellt werden. Ebenso kann vorgesehen sein, dass das Bereitstellen oder Erstellen der ersten und der zweiten spektrometrischen Messdaten vom Test-Zellsubstrat zeitgleich erfolgt. Es kann vorgesehen sein, dass die erste und die zweite Aufbereitung zeitgleich bereitgestellt werden und die spektrometrischen Messdaten der ersten Aufbereitung und der zweiten Aufbereitung zeitgleich bereitgestellt oder erstellt werden. Es kann auch vorgesehen sein, dass die erste und die zweite Aufbereitung zeitgleich bereitgestellt werden und die spektrometrischen Messdaten der ersten und die spektrometrischen Messdaten der zweiten Aufbereitung nicht zeitgleich bereitgestellt oder erstellt werden, wobei typischerweise die spektrometrischen Messdaten der ersten Aufbereitung vor den spektrometrischen Messdaten der zweiten Aufbereitung bereitgestellt oder erstellt werden. Es kann aber auch vorgesehen sein, dass die erste Aufbereitung und die zweite Aufbereitung nicht zeitgleich bereitgestellt werden, wobei typischerweise die erste Aufbereitung vor der zweiten Aufbereitung bereitgestellt wird. Es kann auch vorgesehen sein, dass die erste Aufbereitung und die zweite Aufbereitung nicht zeitgleich bereitgestellt werden und die spektrometrischen Messdaten der ersten und zweiten Aufbereitung zeitgleich bereitgestellt oder erstellt werden, wobei typischerweise die erste Aufbereitung vor der zweiten Aufbereitung bereitgestellt wird. Es kann auch vorgesehen sein, dass die erste Aufbereitung und die zweite Aufbereitung nicht zeitgleich bereitgestellt werden und die spektrometrischen Messdaten der ersten und zweiten Aufbereitung nicht zeitgleich bereitgestellt oder erstellt werden, wobei typischerweise die erste Aufbereitung vor der zweiten Aufbereitung bereitgestellt wird und/oder die spektrometrischen Messdaten der ersten Aufbereitung vor den spektrometrischen Messdaten der zweiten Aufbereitung bereitgestellt oder erstellt werden.

Ebenso wie die erste Aufbereitung kann auch die zweite Aufbereitung einen Vermehrungsschritt des Test-Zellsubstrats umfassen. Nicht deckungsgleich kann hier bedeuten, dass die zweite Aufbereitung in Gegenwart eines wachstumsbeeinflussenden Faktors, z.B. einer bioaktiven Substanz, die die Vitalität und/oder Vermehrungsfähigkeit des Test-Zellsubstrats negativ oder positiv beeinflusst, ausgeführt wird. Die erste Aufbereitung kann hingegen als Wachstumskontrolle ohne Verwendung eines wachstumsbeeinflussenden Faktors oder jedenfalls mit einer abweichenden Konzentration des wachstumsbeeinflussenden Faktors (Verdünnungsreihe) ausgeführt werden und sich damit von den Bedingungen der zweiten Aufbereitung in einem Aspekt unterscheiden, bei ansonsten aber übereinstimmenden Vermehrungsbedingungen. Vermehrungsbedingungen können umfassen: z.B. Typ und Menge eines verwendeten Nährmediums, Zeitraum der Bebrütung, Umgebungsbedingungen wie Temperatur, Zusammensetzung der Umgebungsluft und Feuchte während der Bebrütung und dergleichen.

Das Bereitstellen oder Erstellen spektrometrischer Messdaten kann eine massenspektrometrische Analyse aufweisen, beispielsweise unter Verwendung einer desorbierenden Ionisierung von Biomasse des Test-Zellsubstrats nach erfolgter Aufbereitung, weiter gefolgt von einer massendispergierenden Flugzeitanalyse der erzeugten Ionen, insbesondere eine MALDI-Flugzeitanalyse (MALDI-TOF).

Ebenso wie die erste Aufbereitung kann die zweite Aufbereitung unmittelbar auf einem Probenträger erfolgen, der als Substrat für das Bereitstellen oder Erstellen spektrometrischer Messdaten dient, z.B. einem MALDI-Probenträger wie AnchorChip^{™} (Bruker) oder MBT Biotarget^{™} (Bruker) für massenspektrometrische Messungen oder einem Objektträger aus Glas oder Keramik. Neben erster Aufbereitung und zweiter Aufbereitung können weitere Aufbereitungen des Test-Zellsubstrats unter Bedingungen durchgeführt werden, die mit denen der ersten Aufbereitung und zweiten Aufbereitung sowie untereinander nicht deckungsgleich sind. In diesem Fall können die Messergebnisse der weiteren Aufbereitungen neben einer ersten Teilbibliothek auch mit einer zweiten Teilbibliothek oder weiteren Teilbibliotheken abgeglichen werden. Eine weitere, z.B. dritte Teilbibliothek, kann Referenzdatensätze der Bibliothek umfassen, für die das erste Abgleichergebnis als keine taxonomische Einordnung insbesondere der Art oder Gattung erlaubend bewertet wird und das zweite Abgleichergebnis als eine taxonomische Einordnung erlaubend bewertet wird. Es ist möglich, erste Aufbereitung, zweite Aufbereitung und gegebenenfalls weitere Aufbereitungen auf dem gleichen Probenträger durchzuführen, gegebenenfalls zeitgleich. Ein Beispiel wäre eine Testreihe zur Reaktion eines Test-Zellsubstrats auf verschiedene wachstumsbeeinflussende Faktoren, z.B. bioaktive Substanzen, möglicherweise bei unterschiedlichen Konzentrationen. Ein Beispiel wäre die Bestimmung einer minimalen Hemmkonzentration (MHK) unterschiedlicher antimikrobieller Mittel für einen Test-Mikroorganismus als Test-Zellsubstrat. Ein weiteres Beispiel wäre eine Verdünnungsreihe für eine bioaktive Substanz, z.B. für die Bestimmung einer mittleren inhibitorischen Konzentration (IC₅₀) eines antitumor Faktors, für primäre Tumorzellen oder Tumorzelllinien als Test-Zellsubstrat.

Das Verfahren umfasst überdies: Abgleichen der zweiten spektrometrischen Messdaten oder davon abgeleiteter Daten mit der Teilbibliothek, um ein zweites Abgleichergebnis zu ermitteln. Ferner kann ein Abgleichen der zweiten spektrometrischen Messdaten oder davon abgeleiteter Daten mit einer ersten Teilbibliothek, um ein zweites Abgleichergebnis zu ermitteln, und ein Abgleichen der zweiten spektrometrischen Messdaten oder davon abgeleiteter Daten mit einer zweiten Teilbibliothek, um ein drittes Abgleichergebnis zu ermitteln, vorgesehen sein. In einer Ausführungsform kann die zweite Teilbibliothek diejenigen Referenzdatensätze des ersten Abgleichergebnisses umfassen, die als keine taxonomische Einordnung (z.B. der Art oder Gattung) erlaubend bewertet werden. Die zweite Teilbibliothek kann aber auch aus denjenigen Referenzdatensätzen des ersten Abgleichergebnisses bestehen, die als keine taxonomische Einordnung insbesondere der Art oder der Gattung erlaubend bewertet werden. Ein drittes Abgleichergebnis kann als interne Prozesskontrolle verwendet werden, wenn die zweite Teilbibliothek diejenigen Referenzdatensätze aus der Bibliothek, für die das erste Abgleichergebnis als keine taxonomische Einordnung insbesondere der Art oder der Gattung des Test-Zellsubstrats erlaubend bewertet wird, umfasst oder daraus besteht. In dieser Ausführungsform kann ein drittes Abgleichergebnis, das nur Ähnlichkeitsmaßzahlen umfasst oder daraus besteht, die als keine taxonomische Einordnung insbesondere der Art oder der Gattung erlaubend bewertet werden, eine taxonomische Einordnung des Test-Zellsubstrats der zweiten Aufbereitung und/oder deren Eigenschaftsbestimmung, als zuverlässig bestätigen. In dieser Ausführungsform kann ein drittes Abgleichergebnis, das als eine taxonomische Einordnung insbesondere der Art oder der Gattung erlaubend bewertet wird, verwendet werden, um die taxonomische Einordnung und/oder die Bestimmung einer Eigenschaft von Interesse des Test-Zellsubstrats als nicht zuverlässig zu identifizieren.

Diese könnte der Fall sein, wenn das Test-Zellsubstrat keine homogene Population darstellt. d.h. wenn mindestens zwei Zellpopulationen unterschiedlicher taxonomischer Einordnung im Test-Zellsubstrat vorliegen. Ein solcher Fall findet sich unter anderem bei einer Mischinfektion. In dieser Ausführungsform kann das Verfahren verwendet werden, um zu bestimmen, ob es eine Eigenschaft des Test-Zellsubstrats ist, dass das Zellsubstrat inhomogen ist und/oder die taxonomische Einordnung der im Test-Zellsubstrat vorliegenden Zellpopulationen ermöglichen. Ist ein Test-Zellsubstrat inhomogen, dann liegen mindestens zwei taxonomisch nicht identische Zellpopulationen im Test-Zellsubstrat vor. Liegt ein inhomogenes Test-Zellsubstrat vor, das zwei voneinander verschiedenen Zellpopulationen aufweist oder auch daraus besteht, können sich diese z.B. auf der taxonomischen Ebene der Unterart, der Art, der Gattung oder einer höheren taxonomischen Ebene voneinander unterscheiden. Ein inhomogenes Zellsubstrat im Sinne dieser Anmeldung liegt insbesondere vor, wenn das Zellsubstrat Zellpopulationen umfasst, die sich auf der taxonomischen Ebene der Art oder einer höheren taxonomischen Ebene unterscheiden. Theoretisch sind auch Mischinfektionen, in denen drei oder mehr voneinander verschiedene Zellpopulationen vorliegen, denkbar, die sich dann auf zwei oder mehr taxonomischen Ebenen voneinander unterscheiden können. Bei klinisch relevanten Mischinfektionen, wie die Aktinomykose oder die Aminkolpitis, bereitet in der Regel eine Mikrobenart A einer zweiten Mikrobenart B, z.B. durch Gewebeveränderungen, den Weg und ermöglicht eine parallele Infektion mit der zweiten Mikrobenart B. In diesem Fall liegen dann im Test-Zellsubstrat zwei Zellpopulationen vor, wovon die eine gemäß der taxonomischen Einordnung der Mikrobenart A und die andere gemäß der taxonomischen Einordnung der Mikrobenart B eingeordnet werden.

In einer Ausführungsform des hierin offenbarten Verfahrens kann eine Inhomogenität des Zellsubstrats festgestellt werden. Eine Inhomogenität des Zellsubstrats kann dabei beim Abgleichen der ersten spektrometrischen Messdaten oder davon abgeleiteter Daten der ersten Aufbereitung mit der Bibliothek, um ein erstes Abgleichergebnis zu ermitteln, bestimmt werden. Dies kann der Fall sein, wenn das erste Abgleichergebnis zwei oder mehr Referenzdatensätze enthält, die als eine taxonomische Einordnung des Test-Zellsubstrats erlaubend bewertet werden, wobei die taxonomische Einordnung der Referenzdatensätze nicht deckungsgleich ist, d.h. sich auf einer taxonomischen Ebene wie der Unterart, der Art oder der Gattung oder einer höheren taxonomischen Ebene unterschiedlich ist. Alternativ oder ergänzend kann eine Inhomogenität des Test-Zellsubstrats bestimmt werden, wenn nach einem ersten Abgleichergebnis eine erste Teilbibliothek erstellt wird, beinhaltend Referenzdatensätze aus der Bibliothek, für die das erste Abgleichergebnis als eine taxonomische Einordnung des Test-Zellsubstrats erlaubend bewertet wird, und eine zweite Teilbibliothek erstellt wird, beinhaltend diejenigen Referenzdatensätze aus der Bibliothek, für die das erste Abgleichergebnis als keine taxonomische Einordnung insbesondere der Art oder der Gattung des Test-Zellsubstrats erlaubend bewertet wird.

Weiterhin ist in dieser Ausführungsform vorgesehen, dass die zweiten spektrometrischen Messdaten oder davon abgeleiteten Daten mit der zweiten Teilbibliothek abgeglichen werden, um ein drittes Abgleichergebnis zu erzielen, wobei das dritte Abgleichergebnis Referenzdatensätze beinhaltet, die als eine taxonomische Einordnung erlaubend bewertet werden. Insbesondere kann es in dieser Ausführungsform vorgesehen sein, dass die Bedingungen der zweiten Aufbereitung mit denen der ersten Aufbereitung nicht deckungsgleich sind in mindestens einem Aspekt, der das Bereitstellen oder Erstellen zweiter spektrometrischer Messdaten vom Test-Zellsubstrat einer zweiten Aufbereitung erlaubt, wobei der Aspekt so gewählt ist, dass bevorzugt spektrometrische Messdaten der Zellpopulation, die gemäß dem ersten Abgleichergebnis nicht taxonomisch eingeordnet werden konnte, in der zweiten Aufbereitung gewonnen werden. Dies kann unter anderem dadurch geschehen, dass ein Selektionsdruck auf die Zellpopulation aufgebaut wird, die gemäß dem ersten Abgleichergebnis taxonomisch eingeordnet werden konnte. Ein solcher Selektionsdruck kann dazu führen, dass die Biomasse der Zellpopulation, für die eine taxonomische Einordnung basierend auf dem ersten Abgleichergebnis getroffen werden konnte, in der zweiten Aufbereitung reduziert wird. Dies kann dadurch erreicht werden, dass die zweite Aufbereitung einen wachstumsbeeinflussenden Faktor umfasst, der die Zellpopulation, die in der ersten Aufbereitung taxonomisch eingeordnet werden konnte, abtötet oder ein Wachstum der Zellpopulation, für die eine taxonomische Einordnung basierend auf dem ersten Abgleichergebnis getroffen werden konnte, in der zweiten Aufbereitung reduziert. In dieser Ausführungsform kann es insbesondere vorgesehen sein, dass die zweiten spektrometrischen Messdaten und die zweite Aufbereitung des Test-Zellsubstrats nach dem Abgleichen der ersten spektrometrischen Messdaten mit der Bibliothek, um ein erstes Abgleichergebnis zu ermitteln, bereitgestellt oder erstellt werden. In dieser Ausführungsform kann vorgesehen sein, neben der Bestimmung einer Inhomogenität des Test-Zellsubstrats auch die taxonomischen Einordnungen aller in dem inhomogenen Test-Zellsubstrat enthaltenen Zellpopulationen zu bestimmen.

In einer bevorzugten Ausführungsform umfasst das Verfahren zum spektrometrischen Charakterisieren eines Test-Zellsubstrats, wobei das Test-Zellsubstrat inhomogen ist, folgende Schritte:
- Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen,
- Bereitstellen oder Erstellen erster spektrometrischer Messdaten vom Test-Zellsubstrat nach einer ersten Aufbereitung,
- Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Zellsubstrats oder davon abgeleiteten Daten aufgeführt sind,
- Bereitstellen oder Erstellen einer ersten Teilbibliothek beinhaltend Referenzdatensätze aus der Bibliothek, für die das erste Abgleichergebnis als eine taxonomische Einordnung des Test-Zellsubstrats erlaubend bewertet wird,
- Bereitstellen oder Erstellen einer zweiten Teilbibliothek beinhaltend diejenigen Referenzdatensätze aus der Bibliothek, für die das erste Abgleichergebnis als keine taxonomische Einordnung, insbesondere der Art oder der Gattung, des Test-Zellsubstrats erlaubend bewertet wird,
- Bereitstellen oder Erstellen zweiter spektrometrischer Messdaten vom Test-Zellsubstrat nach wenigstens einer zweiten Aufbereitung unter Bedingungen, die mit den Bedingungen der ersten Aufbereitung nicht deckungsgleich sind,
- Abgleichen der zweiten spektrometrischen Messdaten oder davon abgeleiteter Daten mit der ersten Teilbibliothek, um ein zweites Abgleichergebnis zu ermitteln,
- Abgleichen der zweiten spektrometrischen Messdaten oder davon abgeleiteter Daten mit der zweiten Teilbibliothek, um ein drittes Abgleichergebnis zu ermitteln, und
- Bestimmen einer Eigenschaft des Test-Zellsubstrats unter Verwendung des dritten Abgleichergebnisses, wobei Inhomogenität des Test-Zellsubstrats als Eigenschaft bestimmt wird.
In dieser Ausführungsform können insbesondere diejenigen Referenzdatensätze aus der Bibliothek in der ersten Teilbibliothek beinhaltet sein, für die das erste Abgleichergebnis als eine taxonomische Einordnung der Art oder Unterart erlaubend bewertet wird.

In einer anderen Ausführungsform kann ein eine taxonomische Einordnung insbesondere der Art oder der Gattung erlaubendes, drittes Abgleichergebnis auch darauf hinweisen, dass nicht das gleiche Test-Zellsubstrat für die erste und die zweite Aufbereitung verwendet wurde. In dieser Ausführungsform umfasst das erste und das dritte Abgleichergebnis solche Referenzdatensätze, die als eine taxonomische Einordnung des Test-Zellsubstrats erlaubend bewertet werden und das zweite Abgleichergebnis Referenzdatensätze, die als keine taxonomische Einordnung des Test-Zellsubstrats erlaubend bewertet werden. In dieser Ausführungsform können insbesondere diejenigen Referenzdatensätze aus der Bibliothek in der ersten Teilbibliothek beinhaltet sein, für die das erste Abgleichergebnis als eine taxonomische Einordnung der Art oder Unterart erlaubend bewertet wird.

Das Verfahren umfasst auch: Bestimmen einer Eigenschaft des Test-Zellsubstrats unter Verwendung des zweiten Abgleichergebnisses. Für die Bestimmung einer Eigenschaft des Test-Zellsubstrats kann die Bestimmung einer Ähnlichkeitsmaßzahl oder eines Logarithmus einer Ähnlichkeitsmaßzahl, wie eines log(score)s, für das Test-Zellsubstrat nach einer zweiten Aufbereitung nach Abgleich gegen eine Teilbibliothek, ausreichen.

Die zu bestimmende Eigenschaft kann eine Suszeptibilität und/oder Resistenz des Test-Zellsubstrats gegenüber einem wachstumsbeeinflussenden Faktor umfassen. Insbesondere kann die zu bestimmende Eigenschaft eine minimale Hemmkonzentration (MHK) einer bioaktiven Substanz für das Test-Zellsubstrat umfassen. Beispiele für die zu bestimmende Eigenschaft umfassen die MHK eines antimikrobiellen Mittels wie eines Antibiotikums für einen Mikroorganismus, eines Antimykotikums oder der mittleren inhibitorischen Konzentration (IC₅₀) eines antitumor-Wirkstoffs. Die Bestimmung einer Eigenschaft des Test-Zellsubstrats, z.B. bei der Bestimmung einer Resistenz/Suszeptibilität gegenüber einem wachstumsbeeinflussenden Faktor, kann beispielsweise aus dem log(score) des zweiten Abgleichergebnisses geschlossen werden. Ein log(score) im zweiten Abgleichergebnis, der als eine taxonomische Einordnung erlaubend bewertet wird, weist auf eine Resistenz hin, ein log(score) der als keine taxonomische Einordnung insbesondere der Art oder Gattung erlaubend bewertet wird, weist auf eine Suszeptibilität des Test-Zellsubstrats hin. Das Verfahren kann auch umfassen: Bestimmen einer Eigenschaft des Test-Zellsubstrats unter Verwendung eines dritten Abgleichergebnisses, wenn eine erste und eine zweite Teilbibliothek bereitgestellt oder erstellt werden. Eigenschaften des Test-Zellsubstrats, die unter Verwendung eines dritten Abgleichergebnisses bestimmt werden, können die Inhomogenität des Test-Zellsubstrats beinhalten oder, dass nicht das gleiche Test-Zellsubstrat für die erste und zweite Aufbereitung verwendet wurde, z.B. aufgrund einer zwischenzeitlich eingetretenen Verunreinigung oder Kontamination.

Die vorliegende Offenbarung adressiert den Bedarf, ein verbessertes Verfahren für eine spektrometrische Charakterisierung von Zellsubstraten und insbesondere von Mikroorganismen bereitzustellen. Zu dieser Charakterisierung zählen deren taxonomische Einordnung und die Bestimmung von Eigenschaften des Zellsubstrats. Insbesondere verbessert das Verfahren die Spezifität einer spektrometrischen taxonomischen Einordung und die Bestimmung einer Eigenschaft von Zellsubstraten und verbessert insbesondere die Spezifität der taxonomischen Einordnung von Zellsubstraten, insbesondere Mikroorganismen, u. a. durch eine Reduktion von zweiten Abgleichergebnissen, die falsch-positiv als eine taxonomische Einordnung erlaubend bewertet werden, weiter. In zeitkritischen Szenarien wie der klinischen, mikrobiellen Diagnostik ermöglicht das Verfahren eine schnellere Bereitstellung einer verlässlichen, d.h. hohe Spezifität aufweisenden, Bestimmung kritischer Eigenschaften von Test-Zellsubstraten insbesondere Test-Mikroorganismen, wie die Suszeptibilität und/oder Resistenz gegenüber bestimmten wachstumsbeeinflussenden Faktoren, z.B. Antibiotika oder Antimykotika. Dies ermöglicht die schnellere Behandlung von Patienten durch eine verbesserte Qualitätssicherung, da mögliche zeitaufwendige, nachgeschaltete Verfahren zur Bestimmung einzelner, interessierender Eigenschaften des Zellsubstrats nicht mehr erforderlich sind.

Es wurde im Rahmen der Erfindung festgestellt, dass sich durch den Abgleich des spektrometrischen Messergebnisses des Test-Zellsubstrats der zweiten Aufbereitung mit der ersten Teilbibliothek anstelle der gesamten, häufig mehrere tausend Referenzsätze enthaltenden Bibliothek als zweites Abgleichergebnis eine erhöhte Spezifität bei der Bestimmung von mindestens einer Eigenschaft des Test-Zellsubstrats erreichen lässt. Weiterhin wurde im Rahmen der Erfindung erkannt, dass weitere spektrometrische Messergebnisse, bevorzugt massenspektrometrische Messergebnisse, von dem Test-Zellsubstrat (gegebenenfalls aus einem biologischen Replikat) aus einer zweiten Aufbereitung gegen eine zweite Teilbibliothek als interne Prozesskontrolle geprüft werden können. Die zweite Teilbibliothek umfasst dabei vorzugsweise solche Referenzdatensätze der gesamten Bibliothek für die das erste Abgleichergebnis als keine taxonomische Einordnung insbesondere der Art oder Gattung erlaubend bewertet wird. Das daraus resultierende dritte Abgleichergebnis erlaubt u.a. Rückschlüsse, ob bei der Prozessierung des Test-Zellsubstrats aus der zweiten Aufbereitung möglicherweise ein Fehler aufgetreten ist und das Test-Zellsubstrat vertauscht wurde, oder ob das Test-Zellsubstrat nicht aus einer homogenen Population besteht oder zwischen erster und zweiter Aufbereitung unabsichtlich verunreinigt wurde.

### Kurze Beschreibung der Abbildungen

Zum besseren Verständnis der Erfindung wird auf die folgenden Abbildungen verwiesen. Die Elemente in den Abbildungen sind nicht unbedingt maßstabsgetreu dargestellt, sondern sollen in erster Linie die Prinzipien der Erfindung (größtenteils schematisch) veranschaulichen. In den Abbildungen sind einander entsprechende Elemente in den verschiedenen Ansichten durch gleiche Bezugszeichen gekennzeichnet.

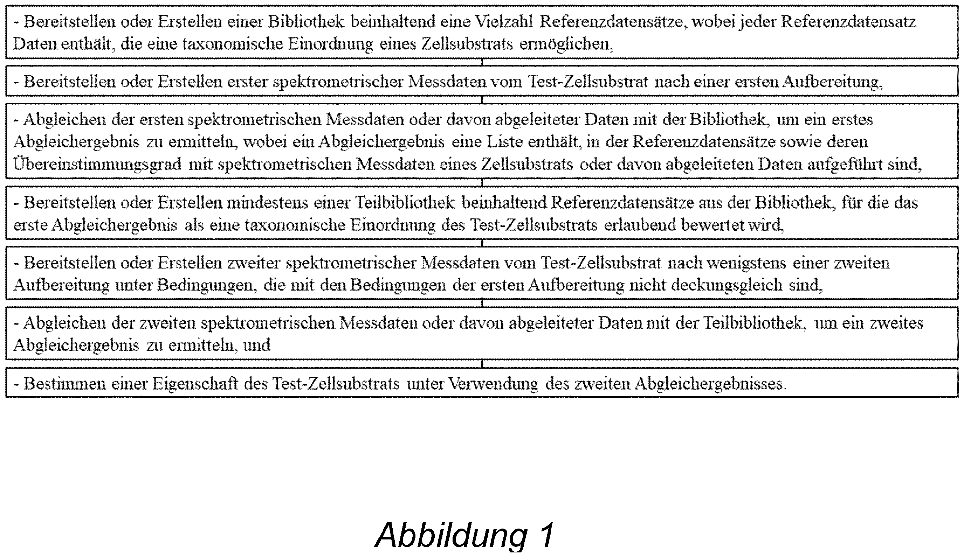

Abbildung 1 zeigt ein Diagramm der wesentlichen Verfahrensschritte zum spektrometrischen Charakterisieren eines Test-Zellsubstrats.

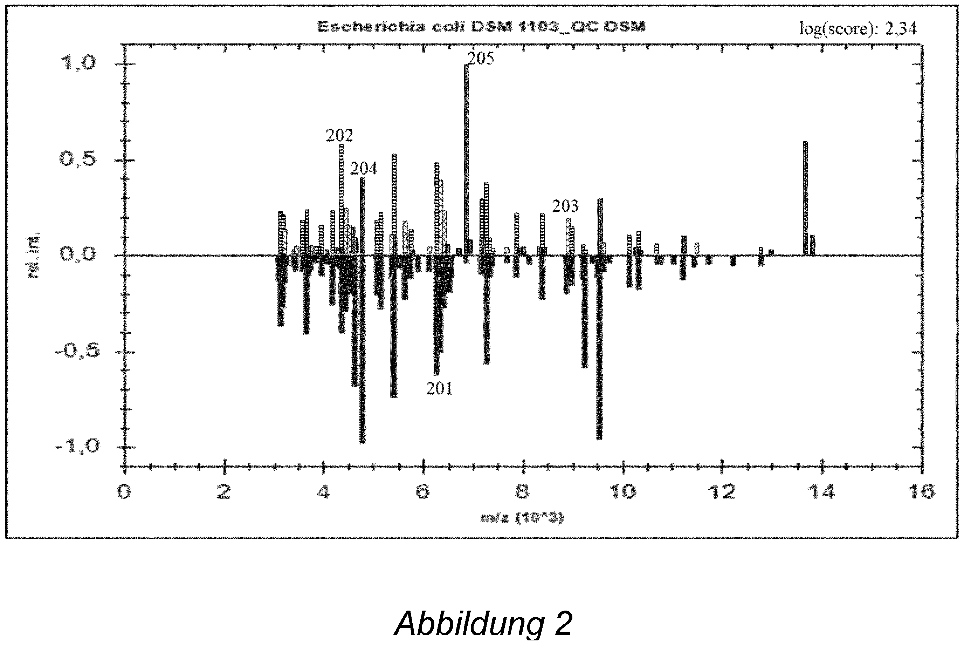

Abbildung 2 zeigt ein Beispiel für eine massenspektrometrische Analyse einer ersten Aufbereitung, die als Wachstumskontrolle angelegt war, und den Abgleich der massenspektrometrischen Daten mit einer relevanten Referenzbibliothek aus dem MALDI Biotyper^{®} zur Identifizierung des Test-Zellsubstrats *E*. *coli.* Der höchste Übereinstimmungsgrad der gemessenen massenspektrometrischen Daten mit aus der Bibliothek gewählten Referenzdatensätzen **201** wurde in Form des Logarithmus der Ähnlichkeitsmaßzahl (log(score)) von 2,34 für ein massenspektrometrisches Referenzspektrum von *E*. *coli* bestimmt. Die taxonomische Einordnung des Test-Zellsubstrats durch Abgleich des Massenspektrums mit der Referenzbibliothek erfolgte somit zuverlässig als *E*. *coli.*

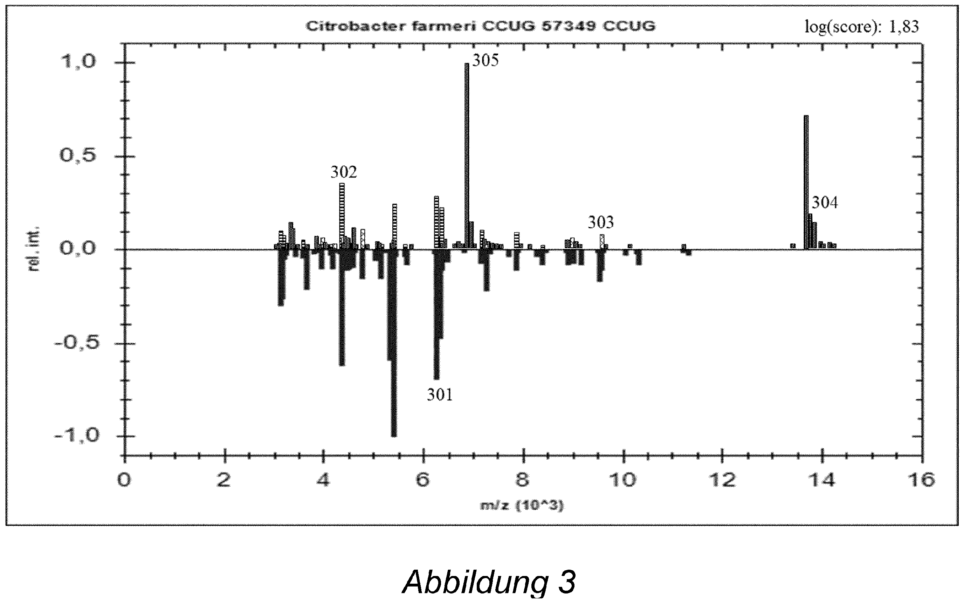

Abbildung 3 zeigt ein Beispiel für eine massenspektrometrische Analyse einer zweiten Aufbereitung des gleichen Test-Zellsubstrats *E*. *coli,* die in einem Cefotaxim (CTX)-haltigen Nährmedium kultiviert wurde ((CTX) Behandlung) wie für Abbildung 2 durchgeführt. Der Abgleich der spektrometrischen Messdaten mit derselben, gesamten Referenzbibliothek, wie für Abbildung 2 verwendet, wurde zur Identifizierung einer Eigenschaft, Resistenz oder Suszeptibilität gegenüber CTX, des Test-Zellsubstrats verwendet. Der höchste Übereinstimmungsgrad der spektrometrischen Messdaten mit aus der Bibliothek gewählten Referenzdatensätzen **301** wurde als Logarithmus der Ähnlichkeitsmaßzahl (log(score)) von 1,83 mit einem massenspektrometrischen Referenzdatensatz von *Citrobacter farmeri* aus der gesamten Referenzbibliothek bestimmt und somit als eine taxonomische Einordnung der Gattung erlaubend bewertet. Im Zuge des zweiten Abgleichergebnisses wurde für das Test-Zellsubstrat mit der taxonomischen Einordnung *Citrobacter farmeri* in der zweiten Aufbereitung die Eigenschaft "CTX-resistent" phänotypisch bestimmt. Die Bestimmung der Eigenschaft des Test-Zellsubstrats durch Abgleich des Linien-Massenspektrums mit der Referenzbibliothek ergab somit die falsch-positive Bestimmung des Test-Zellsubstrats als CTX-resistentes *Citrobacter farmeri.* Ein solches Charakterisierungsergebnis kann von einem Fachmann relativ schnell als unzuverlässig verworfen werden, wenn z.B. die Detektion des Quantifizierungsstandards **305** nicht zuverlässig erfolgte, die taxonomische Einordnung auf einer geringen Anzahl an Massen-Peaks, die im Abgleichergebnis mit hoher Zuverlässigkeit einem Peak des Referenzdatensatzes zugeordnet werden konnte, basierte und/oder die Anzahl an Peaks der Messdaten im verwendeten Referenzdatensatz mit denen das Messergebnis abgeglichen wurde, gering war.

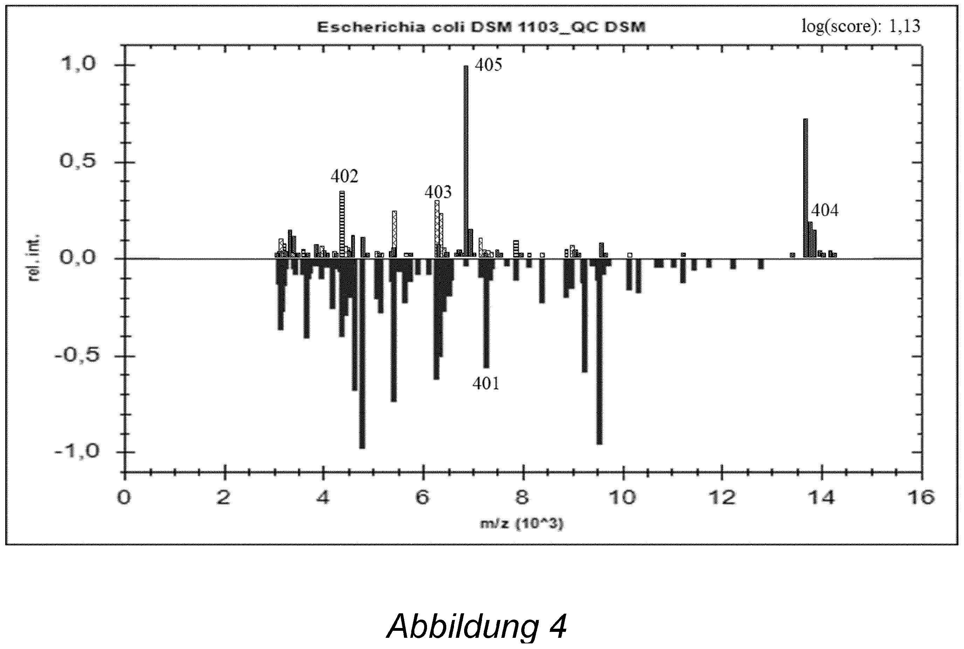

Abbildung 4 zeigt ein Beispiel für eine massenspektrometrische Analyse der zweiten Aufbereitung des gleichen Test-Zellsubstrats *E*. *coli* ((CTX) Behandlung) wie für Abbildung 3 durchgeführt, gemäß der vorliegenden Erfindung. Hierzu wurden vor Erstellen der spektrometrischen Messdaten der zweiten Aufbereitung Teilbibliotheken erstellt. Eine erste Teilbibliothek umfasste die fünf massenspektrometrischen Referenzdatensätze aus der Bibliothek, mit denen das gemessene Massenspektrum der ersten Aufbereitung den höchsten Übereinstimmungsgrad aufwies und die als eine taxonomische Einordnung erlaubend bewertet wurden. Eine zweite Teilbibliothek umfasste solche Referenzspektren, die im ersten Abgleichergebnis für die Wachstumskontrolle einen Logarithmus der Ähnlichkeitsmaßzahl (log(score)) von unter 1,7, d.h. einen Logarithmus der Ähnlichkeitsmaßzahl (log(score)), der typischerweise als keine taxonomische Einordnung der Art oder Gattung erlaubend bewertet wird, mit den massenspektrometrischen Messergebnissen der ersten Aufbereitung ergeben hatten. Der Abgleich der spektrometrischen Messdaten der zweiten Aufbereitung mit der ersten Teilbibliothek wurde zur Bestimmung einer Eigenschaft, Resistenz oder Suszeptibilität gegenüber CTX, des Test-Zellsubstrats verwendet. Der höchste Übereinstimmungsgrad der spektrometrischen Messdaten mit aus der ersten Teilbibliothek gewählten Referenzdatensätzen **401** wurde als Logarithmus der Ähnlichkeitsmaßzahl (log(score)) von 1,13 mit einem massenspektrometrischen Referenzdatensatz von *E*. *coli,* demselben Referenzdatensatz, der auch in Abbildung 2 verwendet wurde, bestimmt und somit als keine taxonomische Einordnung der Art oder Gattung erlaubend bewertet. Im Zuge dieses zweiten Abgleichergebnisses wurde für das Test-Zellsubstrat mit der taxonomischen Einordnung *E. coli* in der zweiten Aufbereitung die Eigenschaft "CTX-suszeptibel" phänotypisch bestimmt. Die Bestimmung der Eigenschaft des Test-Zellsubstrats durch Abgleich des Linien-Massenspektrums mit der ersten Teilbibliothek ergab somit die korrekte spezifische Charakterisierung des Zellsubstrats als CTX-suszeptibles *E*. *coli.* Durch den Abgleich mit der zweiten Teilbibliothek konnte ausgeschlossen werden, dass in der zweiten Aufbereitung ein anderes Test-Zellsubstrat als das in der ersten Aufbereitung inokulierte, z. B. als Folge einer Zellsubstratkontamination, oder ein inhomogenes Test-Zellsubstrat in der ersten und zweiten Aufbereitung vorlag.

### Detaillierte Beschreibung

Während die Erfindung anhand einer Anzahl von Ausführungsformen dargestellt und erläutert wurde, werden Fachleute auf dem Gebiet anerkennen, dass verschiedene Änderungen in Form und Detail daran vorgenommen werden können, ohne vom Umfang der in den beigefügten Patentansprüchen definierten technischen Lehre abzuweichen.

Abbildung 1 zeigt ein Diagramm der wesentlichen Verfahrensschritte zum spektrometrischen Charakterisieren eines Test-Zellsubstrats.

Abbildung 2 zeigt ein schematisches Linien-Massenspektrum einer massenspektrometrischen Messung eines Test-Zellsubstrats einer ersten Aufbereitung, die als Wachstumskontrolle angelegt war. Das mit dem MALDI Biotyper^{®} gemessene Massenspektrum der Wachstumskontrolle wurde gegen alle massenspektrometrischen Referenzdatensätze aus der bereitgestellten Bibliothek, die viele tausend Referenzspektren enthält, abgeglichen. Für einen Referenzdatensatz umfassend ein Massenspektrum von *Escherichia coli* wurde der höchste Übereinstimmungsgrad in Form des Logarithmus der Ähnlichkeitsmaßzahl (log(score)) von 2,34 bestimmt. Die für die Bestimmung des Übereinstimmungsgrads verwendeten Referenzpeaks **201** aus der Referenzbibliothek sind als ausgefüllte Balken im negativen Wertebereich dargestellt (Schmetterlingsdarstellung, *butterfly graph*)*.* Der Übereinstimmungsgrad dieses Abgleichergebnisses wurde als eine taxonomische Einordnung der Art zuverlässig erlaubend bewertet, da der log(score) größer als 2,0 war. Solche Massen-Peaks, die im Abgleichergebnis einem Peak des Referenzdatensatzes mit hoher Zuverlässigkeit zugeordnet werden konnten, sind als horizontal gestrichelte Balken im positiven Wertebereich dargestellt **202,** solche, die noch hinreichend zuverlässig einem Peak des Referenzdatensatzes zugeordnet werden konnten, sind als rautierte Balken im positiven Wertebereich dargestellt **203,** und solche Peaks, die nicht mehr hinreichend einem Peak des Referenzdatensatzes zugeordnet werden konnten, sind als gepunktete Balken im positiven Wertebereich dargestellt **204.** Der verwendete relative Quantifizierungsstandard **205** wurde gemessen, jedoch nicht für eine taxonomische Einordnung verwendet, weshalb er als gepunkteter Balken dargestellt ist.

Als Grundsatzbeweis (*proof of pinciple*) für die Ausführbarkeit des offenbarten Verfahrens wurde ein Cefotaxim suszeptibles *E*. *coli* als Test-Zellsubstrat ausgewählt und der Bestimmung einer Eigenschaft des zu untersuchenden Mikroorganismus unter Verwendung eines MALDI Biotyper^{®} Systems zugeführt. Hierzu wurde die Resistenz/Suszeptibilität des Test-Zellsubstrats gegenüber einer wachstumsbeeinflussenden Substanz, in diesem Beispiel dem Antibiotikum Cefotaxim (CTX), mit einem Verfahren gemäß dem Stand der Technik und dem hierin offenbarten Verfahren untersucht.

Das Test-Zellsubstrat wurde in einem Nährmedium, in Mueller-Hinton Nährmedium (erste Aufbereitung - Abbildung 2) sowie dem gleichen Nährmedium, in dem CTX in einer Konzentration von 1 µg/mL (zweite Aufbereitung - Abbildungen 3 und 4) enthalten war, inkubiert. Die erste Aufbereitung diente als Wachstumskontrolle. Die zweite Aufbereitung wurde verwendet, um die Resistenz/Suszeptibilität des Test-Zellsubstrats gegenüber CTX zu bestimmen.

Für die Erstellung der Aufbereitungen wurde ein großes Volumen (Stocksuspension) einer Zellsuspension, hier etwa 12mL, ansonsten regelmäßig 10 bis 50mL, des Test-Zellsubstrats in Mueller-Hinton Nährmedium, einem flüssigen Nährmedium, hergestellt. Die Konzentration des Test-Zellsubstrats in der Stocksuspension wurde densitometrisch bestimmt, um eine geeignete und zwischen den Aufbereitungen gleiche Menge des Test-Zellsubstrats für die erste und die zweite Aufbereitung zu verwenden. Aus dieser Stocksuspension wurde für eine erste Aufbereitung ein geringes Volumen entnommen und in eine Kavität einer Mikrotiterplatte überführt. Da die erste Aufbereitung als Wachstumskontrolle konzipiert war, befand sich kein Antibiotikum in der Kavität. Parallel wurde für eine zweite Aufbereitung das gleiche Volumen der Stocksuspension entnommen und in eine andere Kavität der Mikrotiterplatte überführt, wodurch das Antibiotikum CTX, das in getrockneter Form in der Kavität der zweiten Aufbereitung vorhanden war, für eine finale Konzentration von 1 µg/mL, gelöst wurde. Nach dem Lösen wurde die Mikrotiterplatte agitiert, wodurch ein vollständiges Lösen und eine homogene Verteilung des Antibiotikums in der Kavität der zweiten Aufbereitung sichergestellt wurde. Weitere Aufbereitungen in Form einer Konzentrations-Verdünnungsreihe zur Bestimmung einer minimalen Hemmkonzentration (MHK) des Antibiotikums CTX können problemlos durch gezieltes Lösen des getrockneten CTX in weiteren Kavitäten der Mikrotiterplatte generiert werden. So kann z.B. in einer dritten Kavität für eine weitere Aufbereitung eine CTX-Konzentration im Mueller-Hinton Nährmedium von 0,5 µg/mL eingestellt werden und in einer vierten Kavität für noch eine weitere Aufbereitung eine CTX-Konzentration von 2 µg/mL. Damit kann die Verdünnungsreihe die CTX-Konzentrationen 0,5, 1 und 2 µg/mL umfassen, weitere Verdünnungsschritte, z.B. 0,25 oder 4 µg/mL können umfasst sein. Eine solche Verdünnungsreihe kann ebenfalls in verschieden großen Verdünnungsschritten der CTX-Konzentration durchgeführt werden.

Anschließend wurde ein geringes Volumen von 6 µL der ersten Aufbereitung des Test-Zellsubstrats, das in Mueller-Hinton Nährmedium vorlag, auf einem Probenpunkt eines MBT Biotarget^{™} Probenträgers platziert. Ebenso wurde für die zweite Aufbereitung ein geringes Volumen von 6 µL der zweiten Aufbereitung des Test-Zellsubstrats, das in 1 µg/mL CTX-haltigem Mueller-Hinton Nährmedium vorlag, auf einem weiteren Probenpunkt des MBT Biotarget^{™} Probenträgers aufgebracht. Weitere Inokula des Test-Zellsubstrats mit anderen CTX-Konzentrationen im Mueller-Hinton Nährmedium (Verdünnungsreihe des Antibiotikums s.o.) können auf weiteren Probenpunkten des Probenträgers aufgebracht werden, um z.B. einen MHK von CTX für das Test-Zellsubstrat spektrometrisch zu bestimmen. Durch die Bestimmung der CTX-Konzentration der Aufbereitung, in der das Wachstum des Test-Zellsubstrats im Vergleich zur ersten Aufbereitung, gerade noch gehemmt wird, würde sich die MHK als Eigenschaft der zweiten Aufbereitung bestimmen lassen. Vorliegend würde die MHK zuverlässig bei 0,5 µg/mL oder 1 µg/mL CTX bestimmt werden können, wenn weitere Aufbereitungen mit 0,5, 1 und 2 µg/mL CTX in der Mikrotiterplatte angesetzt worden wären.

Die MBT Biotarget^{™} Probenträgerplatte wurde in einer Inkubationskammer für 4 Stunden bei konstanten Umweltbedingungen, wie Temperatur und Luftfeuchtigkeit, inkubiert. In Wiederholungen des Versuches wurde die Inkubationszeit auf 6 Stunden angehoben, wodurch die für die Messung zur Verfügung stehenden Biomassen, die von den Mikroorganismen in den Aufbereitungen generiert wurden, vergrößert werden konnten. In dieser Zeit konnte sich das Zellsubstrat an der Schnittstelle zwischen Tropfenflüssigkeit und Trägeroberfläche anlagern bzw. dort sedimentieren. Nach der Stehzeit von 4 Stunden wurde die Restflüssigkeit des Nährmediums vom Probenfleck entfernt mittels eines saugfähigen Gewebes, das seitlich an der Trägeroberfläche mit dem Tropfen auf einem Fleck in Fluidkontakt gebracht wurde und einen Großteil der Flüssigkeit einfach abgesaugt hat. Die auf diese Weise freigelegte Test-Zellsubstratablagerung konnte im Anschluss, wie aus dem Stand der Technik bekannt, weiter präpariert und in einem Massenspektrometer vermessen werden. Zum Beispiel konnten Peptide/Proteine der Zellsubstrate extrahiert und/oder das abgelagerte Zellsubstrat in eine MALDI-Matrixsubstanz eingebettet werden, wobei ein Quantifizierungsstandard der Zellsubstratprobe zusammen mit der Matrix hinzugefügt werden konnte. In den für Abbildungen 2 bis 4 durchgeführten Versuchen wurde ein Quantifizierungsstandard der Test-Zellsubstratprobe hinzugefügt. Der für diesen Quantifizierungsstandard gemessene, alle anderen Massensignale bei weitem überragende Peak wurde jedoch als extrinsischer Peak nicht für die taxonomische Einordnung verwendet und ist daher als gepunkteter Balken im positiven Wertebereich dargestellt (**205, 305, 405**).

Die Test-Zellsubstrate der ersten und der zweiten Aufbereitung wurden anschließend einer massenspektrometrischen Analyse mit dem MALDI Biotyper^{®} System zugeführt. Zunächst wurde die erste Aufbereitung des Test-Zellsubstrats, die als Wachstumskontrolle verwendet wurde, massenspektrometrisch gemessen und eine taxonomische Einordnung des Test-Zellsubstrats durch Abgleich des erhaltenen Linien-Massenspektrums mit den Referenzmassenspektren der Referenzbibliothek des MALDY Biotyper^{®} Systems vorgenommen.

Innerhalb des erhaltenen ersten Abgleichergebnisses wurde das Referenzmassenspektrum mit dem höchsten Übereinstimmungsgrade (*best match*) zum gemessenen Spektrum zur taxonomischen Einordnung verwendet. Abbildung 2 zeigt exemplarisch eine solche beste Übereinstimmung für die erste Aufbereitung. Das gemessene Massenspektrum wies einen Logarithmus der Ähnlichkeitsmaßzahl (log(score)) von 2,34 mit einem Referenzspektrum von *E. coli* DSM 1103 auf. Neben dieser besten Übereinstimmung, d.h. der massenspektrometrische *E. coli* Referenzdatensatz, der dem gemessenen Spektrum am ähnlichsten war, wurden innerhalb des ersten Abgleichergebnisses weitere ähnliche, z.B. eine taxonomische Einordnung bzgl. der Gattung *Escherichia sp.* erlaubende, sowie nicht-ähnliche massenspektrometrische Referenzdatensätze innerhalb der gesamten Bibliothek ermittelt. Die nicht-ähnlichen massenspektrometrischen Referenzdatensätze erlaubten keine taxonomische Einordnung der Art oder der Gattung des Zellsubstrats und gehörten z.B. zu anderen Gattungen wie *Citrobacter sp.* Die Referenzdatensätze aus der Bibliothek, die im ersten Abgleichergebnis aufgeführt wurden, konnten aufgrund des Logarithmus der Ähnlichkeitsmaßzahl als eine taxonomische Einordnung erlaubende oder keine taxonomische Einordnung der Art oder Gattung erlaubende Abgleichergebnisse bewertet und eingeteilt werden. Der Abgleich des erhaltenen Massespektrums der ersten Aufbereitung mit der Referenzbibliothek ordnete das gemessene Linien-Massenspektrum aufgrund des log(score)s von 2,34 der Art *E*. *coli* als dem zu untersuchenden Test-Zellsubstrat zu, da der Wert über dem log(score) 2,0 lag, wie es der Vorgabe des MALDI Biotyper^{®}-Systems entspricht.

Weiterhin wurde die Eigenschaft Suszeptibilität oder Resistenz (Suszeptibilität/Resistenz) gegenüber CTX mit einem Verfahren aus dem Stand der Technik und dem hierin offenbarten Verfahren für die zweite Aufbereitung des Test-Zellsubstrats bestimmt. Die zweite Aufbereitung (CTX-Behandlung) wurde massenspektrometrisch vermessen und das erhaltene Linien-Massenspektrum gegen die gesamte Referenzbibliothek abgeglichen (Abbildung 3). Es wurde eine geringe Anzahl an massenspektrometrischen Peaks gemessen. Auf Basis dieser geringen Anzahl an Peaks wurde der Abgleich mit der gesamten MALDI Biotyper^{®} Bibliothek vorgenommen. In Abbildung 3 ist ein Linien-Massenspektrum, als Teil eines Abgleichergebnisses, einer massenspektrometrischen Messung gezeigt, sowie ein log(score) von 1,82 als Bewertung des Übereinstimmungsgrads mit einem Referenzspektrum von *Citrobacter farmeri* (*best match*) aufgeführt. Die für die Bestimmung des Übereinstimmungsgrads verwendeten Referenzpeaks **301** sind als ausgefüllte Balken im negativen Wertebereich dargestellt. Solche Massen-Peaks, die im Abgleichergebnis einem Peak des Referenzdatensatzes mit hoher Zuverlässigkeit zugeordnet werden konnten, sind als horizontal gestrichelte Balken im positiven Wertebereich dargestellt **302,** solche, die noch hinreichend zuverlässig einem Peak des Referenzdatensatzes zugeordnet werden konnten, sind als rautierte Balken im positiven Wertebereich dargestellt **303,** und solche Peaks, die nicht mehr hinreichend einem Peak des Referenzdatensatzes zugeordnet werden konnten, sind als gepunktete Balken im positiven Wertebereich dargestellt **304.** Der verwendete relative Quantifizierungsstandard **305** wurde gemessen, jedoch nicht für die taxonomische Einordnung verwendet.

Die taxonomische Einordnung basierte auf einem log(score) von 1,83, der als eine zuverlässige Bestimmung der Gattung erlaubend bewertet wird. Daher wurde für die Bestimmung des Eigenschaft Resistenz/Suszeptibilität in Kultivierbarkeit, d.h. Resistenz gegenüber CTX bestimmt, obwohl das Test-Zellsubstrat eigentlich empfindlich (suszeptibel) gegenüber CTX gewesen ist. Eine zuverlässige taxonomische Einordnung spielt für eine phänotypische Bestimmung einer Resistenz oder Suszeptibilität gegenüber einem antimikrobiellen Mittel wie einem Antibiotikum eine wichtige Rolle. Die taxonomische Einordnung des Test-Zellsubstrats als *Citrabacter sp.* trug somit zur Bestimmung des Test-Zellsubstrats als resistent bei.

Im Ergebnis kann es somit vorkommen, dass die (falsch-positive) Bewertung als eine taxonomische Einordnung zu einer Gattung abweichend von der taxonomischen Einordnung der Wachstumskontrolle, wie vorliegend eine taxonomische Einordnung zur Gattung *Citrobacter sp.,* erlaubend und eine Bestimmung der Eigenschaft Resistenz gegenüber CTX für das Zellsubstrat erfolgte. In diesem experimentellen Ansatz kann, muss aber nicht, ein log(score) bestimmt werden, der eine taxonomische Einordnung der Art erlaubt hat oder ein log(score), der eine taxonomische Einordnung der Gattung erlaubt hat, wie Wiederholungsexperimente zeigten und in Abbildung 3 dargestellt ist. Es kann somit vorkommen, dass für die Bestimmung der Resistenzeigenschaft der zweiten Aufbereitung trotz geringer Anzahl an gemessenen Massenpeaks im zweiten Messergebnis oder trotz des Ermittelns des Abgleichergebnisses der erstellten zweiten Messdaten mit der gesamten Referenzbibliothek mit Hilfe einer geringen Anzahl an gemessenen Peaks, die mit hoher **302** oder hinreichender **303** Zuverlässigkeit einem Referenzpeak in den verwendeten Referenzspektren **301** zugeordnet werden konnte, im erhaltenen zweiten Abgleichergebnis ein eine taxonomische Einordnung erlaubendes Abgleichergebnis ermittelt und die Eigenschaft CTX-resistent bestimmt wurde. Diese falsch-positiven Charakterisierungsergebnisse können vom fachkundigen Anwender nach Kontrolle verworfen werden, da z.B. für die phänotypische Bestimmung der Eigenschaft CTX-Suszeptibilität oder Resistenz mittels des gemessenen Massenspektrums der zweiten Aufbereitung, die Anzahl der gemessenen Peaks gering und/oder die Anzahl an gemessenen Peaks, die mit hoher **302** oder hinreichender **303** Zuverlässigkeit einem Referenzpeak in den verwendeten Referenzspektren **301** zugeordnet werden konnte, gering war, was für eine zuverlässige taxonomische Bestimmung bzw. eine Bestimmung der Eigenschaft des Test-Zellsubstrats unzureichend war. Eine geringe Biomasse des Zellsubstrats in der zweiten Aufbereitung nach Ablauf der Inkubationszeit hat zur falsch-positiven Zuordnung CTX-resistentes *Citrobacter sp.* beigetragen.

In einer parallel zu der Charakterisierung des Test-Zellsubstrats in der zweiten Aufbereitung für Abbildung 3 durchgeführten massenspektrometrischen Analyse der massenspektrometrischen Messdaten der zweiten Aufbereitung für Abbildung 4, wurden vor der Erstellung der spektrometrischen Messdaten der zweiten Aufbereitung Teilbibliotheken erstellt. Eine erste Teilbibliothek diente dem Bereitstellen eines zweiten Abgleichergebnisses basierend auf den Messergebnissen der zweiten Aufbereitung. Diese erste Teilbibliothek umfasste die fünf massenspektrometrischen Referenzdatensätze aus der Bibliothek, die dem gemessenen Massenspektrum aus der ersten Aufbereitung am ähnlichsten waren und die als eine taxonomische Einordnung erlaubend bewertet wurden. Bei der Auswahl aus der Bibliothek wurden die fünf Referenzdatensätze, die die höchsten Ähnlichkeitsmaßzahlen ("score") bzw. deren Logarithmus (log(score)) aufwiesen, ausgewählt. Solche Referenzdatensätze, die zu anderen Gattungen als der Gattung, die für das Test-Zellsubstrat aufgrund des Abgleichergebnisses der Messdaten der ersten Aufbereitung bestimmt worden war, wie *Citrobacter sp.,* gehörten, wurden in der ersten Teilbibliothek ausgeschlossen, da sie im ersten Abgleichergebnis keine taxonomische Einordnung erlaubt hatten. In Wiederholungen der Analyse wurde die Anzahl der in der zweiten Teilbibliothek enthaltenen massenspektrometrischen Referenzdatensätze auf die drei Referenzdatensätze mit dem höchsten Übereinstimmungsgrad reduziert, wobei die verbesserte Wirkung beibehalten wurde.

Der Abgleich der spektrometrischen Messdaten der zweiten Aufbereitung mit der ersten Teilbibliothek wurde zur Identifizierung einer Eigenschaft, Resistenz oder Suszeptibilität gegenüber CTX, des Test-Zellsubstrats verwendet. Solche Massen-Peaks, die im Abgleichergebnis einem Peak des Referenzdatensatzes mit hoher Zuverlässigkeit zugeordnet werden konnten, sind als horizontal gestrichelte Balken im positiven Wertebereich dargestellt **402,** solche, die noch hinreichend zuverlässig einem Peak des Referenzdatensatzes zugeordnet werden konnten, sind als rautierte Balken im positiven Wertebereich dargestellt **403,** und solche Peaks, die nicht mehr hinreichend einem Peak des Referenzdatensatzes zugeordnet werden konnten, sind als gepunktete Balken im positiven Wertebereich dargestellt **404.** Der verwendete relative Quantifizierungsstandard **405** wurde gemessen, jedoch nicht für die taxonomische Einordnung verwendet. In dem zweiten Abgleichergebnis aus dem Abgleich der massenspektrometrischen Daten der zweiten Aufbereitung mit der ersten Teilbibliothek wurde der log(score) auf 1,13 (d.h. unter 1,7) mit einem massenspektrometrischen Referenzspektrum **401,** dargestellt als ausgefüllte Balken im negativen Wertebereich, von *E*. *coli,* demselben Referenzdatensatz, der auch in Abbildung 2 verwendet wurde, und damit die Eigenschaft des Zellsubstrats als CTX-suszeptibel bestimmt (Abbildung 4). Damit konnte gezeigt werden, dass das Zellsubstrat *E. coli* gemäß der taxonomischen Einordnung der ersten Aufbereitung in CTX-haltigem Mueller-Hinton Nährmedium nicht überlebt hatte. Das Zellsubstrat wurde somit richtigerweise als CTX-suszeptibles *E. coli* bestimmt. Die Spezifität der taxonomischen Einordnung und der Bestimmung der CTX-Resistenzeigenschaft, selbst bei unzureichender Messdatenlage, wurde somit verbessert und eine manuelle Kontrolle der Abgleichergebnisse der zweiten Aufbereitung überflüssig gemacht bzw. deren Notwendigkeit zumindest stark reduziert. Dadurch wurde die Spezifität der Charakterisierung des Test-Zellsubstrats verbessert und die Möglichkeit für eine Automatisierung der Charakterisierung des Test-Zellsubstrats verbessert, da die Notwendigkeit für manuelle Kontrolle der Bestimmung der Eigenschaft des Test-Zellsubstrats überflüssig, zumindest jedoch stark reduziert werden kann.

Neben der ersten Teilbibliothek wurde eine zweite Teilbibliothek erstellt. Die zweite Teilbibliothek umfasste dabei solche Referenzspektren, die im ersten Abgleichergebnis für die Wachstumskontrolle einen Logarithmus der Ähnlichkeitsmaßzahl (log(score)) von unter 1,7, d.h. typischerweise bewertet als keine taxonomische Einordnung der Art oder Gattung erlaubend, mit den massenspektrometrischen Messergebnissen der ersten Aufbereitung ergeben hatten. Insbesondere waren die fünf massenspektrometrischen Referenzdatensätze der ersten Teilbibliothek nicht in der zweiten Teilbibliothek umfasst. Die zweite Teilbibliothek umfasste diejenigen Referenzdatensätze die im ersten Abgleichergebnis als keine taxonomische Einordnung der Art oder Gattung erlaubend bewertet wurden. Die spektrometrischen Messergebnisse der zweiten Aufbereitung wurden, neben dem Abgleich mit der ersten Teilbibliothek ebenfalls mit der zweiten Teilbibliothek abgeglichen. Dieser dritte Abgleich diente der Qualitätssicherung des zweiten Abgleichergebnisses in Form einer Negativkontrolle. Ein eine taxonomische Einordnung erlaubendes Ergebnis sollte in diesem dritten Abgleich nicht erzielt werden, sollte sogar ausgeschlossen sein. Im durchgeführten Versuch konnte für dieses dritte Abgleichergebnis kein eine taxonomische Einordnung erlaubender Logarithmus der Ähnlichkeitsmaßzahl bestimmt werden. Dadurch konnte ausgeschlossen werden, dass evtl. ein anderes Test-Zellsubstrat als das ursprünglich inokulierte als Folge einer Zellsubstratkontamination oder ein inhomogenes Test-Zellsubstrat in der zweiten Aufbereitung vorlag.

### Definitionen

Soweit nicht anderweitig definiert, umfassen die verwendeten Formulierungen das allgemeine und technische Verständnis des Fachmanns. Insbesondere beschreiben die folgenden Formulierungen das technische Verständnis des Fachmanns. Beispiele sind nicht zur Beschränkung der Erfindung vorgesehen, sondern zur Erläuterung des Verständnisses des Fachmanns.

Soweit "ein", "eine", "der", "die" oder "das" hier verwendet werden, kann damit eins oder auch mehrere gemeint sein. Beispielsweise kann "eine Zelle" eine einzelne Zelle oder auch eine Vielzahl von Zellen beschreiben.

Soweit "und/oder" hier verwendet wird, bezieht es sich auf und umfasst jedes und jede mögliche Kombination von einem oder mehreren assoziierten aufgelisteten Gegenständen, sowie das Fehlen von Kombinationen, wenn es als Alternative, d.h. als "oder", verknüpft ist.

Des Weiteren bedeuten Begriffe wie "ca.", "etwa" o.ä., wenn sie sich auf eine messbare Größe beziehen wie z.B. die Menge eines Agens, wie einem wachstumsbeeinflussenden Faktor, dass Variationen dieser Größe von ±20%, ±10%, ±5%, ±1%, ±0,5% oder auch ±0,1% umfasst sind.

Der Begriff "Zellsubstrat" oder "Test-Zellsubstrate" im Sinne dieser Anmeldung beschreibt eine zelluläre Probe, die mit Hilfe spektrometrischer Messungen und Abgleichen gegen Bibliotheken und/oder Teilbibliotheken, charakterisiert werden soll. Dabei beschreibt der Begriff zelluläre Proben, die im Labor kultiviert, vermehrt und/oder im Labor gehandhabt werden können. Dabei umfasst der Begriff prokaryotische und/oder eukaryotische Zellen. Ferner umfasst der Begriff zelluläre Proben, die einen intrazellulären und/oder extrazellulären Lebensstil haben. Der Begriff kann Pflanzen-, Tier und/oder Pilzzellen beschreiben. Weiterhin umfasst der Begriff einzellige und/oder mehrzellige und/oder bewegliche, z.B. begeißelte, und/oder unbewegliche Zellsubstrate. Ein Zellsubstrat kann ein aus einem Organismus isoliertes Zellsubstrat sein. Beispiele für isolierte Zellsubstrate umfassen Tumorzellen, von einem Pathogen befallene Zellen oder bestimmte Zellpopulationen, wie Makrophagen oder T Zellen. Tumorzellen können beispielsweise aus einem Lymphom, einer Leukämie oder einem soliden Tumor isoliert werden. Zellsubstrate können mit verschiedenen Verfahren isoliert werden. Beispiele sind dem Fachmann bekannt und umfassen Durchflusszytometrische Verfahren oder *bead*-basierte Verfahren.

In einer Ausführungsform beschreibt der Begriff "Zellsubstrat" zelluläre Proben, die einen intrazellulären Lebensstil haben, insbesondere intrazelluläre Mikroben, wie *Mycobacterium avium, Mycobacterium intracellulare* oder *Listeria monocytogenes.*

In einer bevorzugten Ausführungsform beschreibt der Begriff "Zellsubstrat" Mikroorganismen" oder auch "Mikroben". Der Begriff "Mikroorganismen" oder "Mikroben" umfasst mikroskopisch kleine Organismen, die aus einer oder wenigen Zellen bestehen. Mikroben umfasst gram-negative und gram-positive Bakterien, Hefen, Schimmelpilze, Parasiten und Mollicutes. Beispiele von gram-negativen Bakterien umfassen Bakterien der folgenden Gattungen: *Pseudomonas, Escherichia, Salmonella. Shigella, Enterobacter, Klebsiella, Serratia, Proteus, Campylobacter, Haemophilus, Morganella, Vibrio, Yersinia, Acinetobacter, Stenotrophomonas, Brevundimonas, Ralstonia, Achromobacter, Fusobacterium, Prevotella, Branhamella, Neisseria, Burkholderia, Citrobacter, Hafnia, Edwardsiella, Aeromonas, Moraxella, Brucella, Pasteurella, Providencia,* und *Legionella.* Beispiele von gram-positiven Bakterien umfassen Bakterien der folgenden Gattungen: *Enterococcus, Streptococcus, Staphylococcus, Bacillus, Paenibacillus, Lactobacillus, Listeria, Peptostreptococcus, Propionibacterium, Clostridium, Bacteroides, Gardnerella, Kocuria, Lactococcus, Leuconostoc, Micrococcus, Mycobacteria und Cornybacteria.* Beispiele von Pilzen umfassen Hefen und Schimmelpilze der folgenden Gattungen: *Candida, Cryptococcus, Nocardia, Penicillium, Alternaria, Rhodotorula, Aspergillus, Fusarium, Saccharomyces* und *Trichosporon.* Beispiele von Parasiten umfassen Parasiten der folgenden Gattungen: *Trypanosoma, Babesia, Leishmania, Plasmodium, Wucheria, Brugia, Onchocerca* und *Naegleria.* Beispiele von Mollicutes umfassen Mollicutes der folgenden Gattungen: *Mycoplasma* und *Ureaplasma.*

Unter dem Singular "Mikrobe" wird, wie im allgemeinen Sprachgebrauch üblich, neben einer einzelnen Mikrobenzelle auch die Mikrobenart verstanden. Unter dem Plural "Mikroben" werden die der Untersuchung unterliegenden Mikrobenzellen verstanden. Mikroorganismen finden sich in den taxonomischen Domänen der Bakterien (Bacteria), der Archeen (Archaea) und der Eukaryoten, und umfasst insbesondere die Bakterien, die Archeen, die Pilze, Mikroalgen und die Protozoen.

In einer bevorzugten Ausführungsform beschreibt der Begriff "Zellsubstrat" oder "Test-Zellsubstrat" Mikroorganismen, bevorzugt Bakterien, besonders bevorzugt gram-negative Bakterien, am meisten bevorzugt Bakterien der Familie der *Enterobacteriaceae.*

In einer weiteren bevorzugten Ausführungsform beschreibt der Begriff "Zellsubstrat" oder "Test-Zellsubstrat" isolierte Tumorzellen, besonders bevorzugt isolierte, maligne Tumorzellen, am meisten bevorzugt isolierte, maligne Tumorzellen eines soliden Tumors.

Der Begriff "Aufbereitung" im Sinne dieser Anmeldung kennzeichnet einen Arbeitsablauf, mit dem die anfangs verfügbare Biomasse des zu untersuchenden Test-Zellsubstrats verarbeitet und für eine spektrometrische Messung, insbesondere eine massenspektrometrische Messung, vorbereitet wird. Eine Aufbereitung kann dabei einen Vermehrungsschritt des Test-Zellsubstrats enthalten, um die verfügbare Biomasse zu vergrößern und damit die nachweisbaren Spektralsignale gegenüber allgegenwärtigem Hintergrund oder Rauschen in spektrometrischen Messdaten stärker hervorzuheben. Dem Fachmann sind verschiedene Formen der Aufbereitung zur Vorbereitung von Zellsubstraten für spektrometrische Messungen bekannt. Eine Aufbereitung kann die Inkubation des Zellsubstrats umfassen.

Der Begriff "Inkubation" im Sinne dieser Anmeldung umfasst alle Formen des Bebrütens, d.h. der Anzucht oder Kultivierung, von Zellsubstraten. Das Zellsubstrat kann dabei einem Vermehrungsschritt unterzogen werden. Eine Inkubation wird ermöglicht durch Schaffung und Aufrechterhaltung von Bedingungen, die ein Leben, Überleben und/oder Wachstum der Test-Zellsubstrate gewährleisten. Die Verwendung von Nähr- oder Kulturmedien, kurz Medien, dient der Inkubation der Test-Zellsubstrate. Typischerweise enthält ein Nährmedium einen Hauptanteil Wasser, einer für das Test-Zellsubstrat verwertbaren Energiequelle und vom Test-Zellsubstrat benötigten Nährstoffen oder Substrate, ein Nährmedium kann aber auch anders zusammengesetzt sein. Weiterhin kann ein Nährmedium Salze enthalten, die dem Organismus wichtige Ionen liefern können, Farbstoffe oder deren Vorstufen, Geliermittel zur Verfestigung des Nährmediums, wie Agar, Gellan und/oder Kieselgel, wachstumsbeeinflussende Faktoren, Indikatoren und/oder Puffersubstanzen. Handelt es sich bei dem Test-Zellsubstrat um eukaryotische, d.h. tierische oder pflanzliche Zellen, wird die Inkubation auch als Zellkultur bezeichnet. Der Begriff "Inkubation" umfasst die Inkubation auf festen, gelierten, halbfesten und/oder in flüssigen Medien, wobei die Medien in verschiedenen Gefäßen oder auf verschiedenen Trägern, wie einem MALDI-Probenträger, z.B. einem AnchorChip^{™} (Bruker) oder einem MBT Biotarget^{™} (Bruker) aufgebracht sein können. Für die Inkubation tierischer Zellen werden vor allem flüssige Nährmedien verwendet, für pflanzliche Zellen vor allem flüssige und feste Nährmedien. Typischerweise wird das Test-Zellsubstrat in einem Medium mit der gleichen Zusammensetzung für die erste und die zweite und/oder die weiteren Aufbereitungen inkubiert. Die Zusammensetzung der Medien für die erste und die zweite und/oder die weiteren Aufbereitungen kann aber auch unterschiedlich sein, wenn der unterschiedliche Inhaltsstoff oder die unterschiedlichen Inhaltsstoffe als wachstumsbeeinflussender Faktor vorgesehen sind. Typischerweise werden Zellsubstrate auf oder in einem Medium in einem Wärmeschrank, Wärmeraum, Inkubator oder Brutschrank inkubiert. Typische Inkubationsdauern betragen 2h, 4h, 6h, 8h, 12h, 24h, 36h oder 48h, bevorzugte Inkubationsdauern betragen 2h bis 8h, bevorzugt 4h bis 6h. Soll das Zellsubstrat einem Vermehrungsschritt unterzogen werden, kennt der Fachmann typische Generationszeiten, d.h. die Zeitdauer, in der sich die Zahl der Individuen einer Population verdoppelt, z.B. ca. 20 Minuten für *E*. *coli,* ca. 30 Minuten für *S. aureus* oder Salmonellen oder ca. 18h bei *Mycobacterium tuberculosis.*

Eine "taxonomische Einordnung" eines "Zellsubstrats" und insbesondere eines Mikroorganismus im Sinne dieser Anmeldung umfasst die Einordnung des Test-Zellsubstrats auf eine taxonomische Ebene bis herunter zur Gattung (Genus), Art (Spezies), Unterart (Subspezies) und/oder der Varietät oder des Serotyps. In einer bevorzugten Ausführungsform umfasst der Begriff die taxonomische Einordnung des Test-Zellsubstrats auf der Ebene der Gattung (Genus) oder Art (Spezies).

Der Begriff "wachstumsbeeinflussender Faktor" im Sinne dieser Anmeldung umfasst jede Substanz, jede Behandlung und/oder jede Umgebungsbedingungen deren Beigabe oder Änderung, die Wachstumsbedingungen für das Test-Zellsubstrat ändern. Wachstumsbeeinflussende Faktoren können eine positive oder eine negative Auswirkung auf die Vitalität und/oder Vermehrungsfähigkeit des Test-Zellsubstrats haben.

Beispiele für wachstumsbeeinflussende Faktoren, die eine positive Auswirkung auf die Vitalität und/oder Vermehrungsfähigkeit des Test-Zellsubstrats haben können sind Wachstumsfaktoren, z.B. hämatopoetische Wachstumsfaktoren wie Erythropoetin oder Granulozyten-koloniestimulierender Faktor (G-CSF), Makrophagen-Kolonien-stimulierender Faktor (M-CSF), Granulozyten-Makrophagen-Kolonien-stimulierender Faktor (GM-CSF), oder für Bakterien durch das Anbieten bestimmter Kohlenstoff, Stickstoff und Schwefelquellen oder das Vorhandensein bestimmter elektromagnetischer Strahlung (Licht) bei (fakultativ) phototrophen Bakterien.

Beispiele für wachstumsbeeinflussende Faktoren, die eine negative Auswirkung auf die Vitalität und/oder Vermehrungsfähigkeit des Test-Zellsubstrats haben können, sind beispielsweise bioaktive Substanzen, insbesondere jeder chemische Stoff (Element, Verbindung oder Gemisch), der unmittelbar toxisch, z.B. bakterizid oder cytotoxisch, oder wachstumshemmend, d.h. bakteriostatisch oder cytostatisch auf das Test-Zellsubstrat wirkt und dessen Vitalität und/oder Wachstum im Vergleich zu einer Wachstumskontrolle, in der dieser chemische Stoff nicht zugesetzt wird, negativ beeinflusst. Die festen, gelierten, halbfesten und flüssigen Medien in oder auf denen eine Inkubation des Test-Zellsubstrats, insbesondere eine Inkubation für eine erste Aufbereitung, stattfindet sind im Sinne dieser Anmeldung keine wachstumsbeeinflussenden Faktoren, denn bei diesen handelt es sich erst um die geschaffenen Bedingungen, die eine Inkubation ermöglichen. Sind diese Bedingungen jedoch nicht deckungsgleich für die Inkubation des Test-Zellsubstrats für eine erste Aufbereitung und eine weitere Aufbereitung, insbesondere einer zweiten Aufbereitung, dann kann der Unterschied in den geschaffenen Bedingungen ein wachstumsbeeinflussender Faktor sein. Beispiele wachstumsbeeinflussender Faktoren umfassen chemische Stoffe, wie antimikrobielle Mittel, wie Antibiotika oder Antimykotika oder Cytostatika.

Ein wachstumsbeeinflussender Faktor kann auch die Kombination mehr als eines Faktors sein, beispielsweise eine Kombination zweier verschiedener Antibiotika. Ferner umfasst der Begriff "wachstumsbeeinflussender Faktor" auch Änderungen der Kultivierungs- und Bebrütungskonditionen, wie Änderungen an der Zusammensetzung eines verwendeten Nährmediums, am Zeitraum der Bebrütung, an den Umgebungsbedingungen wie Temperatur, Zusammensetzung der Umgebungsluft und Feuchte während der Bebrütung. Weiterhin sind vom Begriff "wachstumsbeeinflussender Faktor" auch physikalische Behandlungen des Zellsubstrats umfasst, wie die Bestrahlung des Zellsubstrats mit einem Licht einer bestimmten Wellenlänge oder Intensität. Der wachstumsbeeinflussende Faktor kann bereits vor der Zugabe eines Zellsubstrat-Inokulums im Nährmedium enthalten sein, beispielsweise in Form einer Lösung, eines Pulvers, oder in einer lyophilisierten Form. Der wachstumsbeeinflussende Faktor kann auch in einem Gefäß, wie der Kavität einer Mikrotiterplatte in Form eines Pulvers, in getrockneter oder lyophilisierter Form vorhanden sein und erst durch Zugabe eines bestimmten Volumens des Zellsubstrat-Inokulums gelöst und auf die gewünschte Konzentration eingestellt werden. Alternativ können der oder die wachstumsbeeinflussenden Faktoren dem Nährmedium nach dem Eintragen des Zellsubstrats beigemengt werden. In einer bevorzugten Ausführungsform hat der wachstumsbeeinflussende Faktor eine negative Auswirkung auf die Vitalität und/oder Vermehrungsfähigkeit des Test-Zellsubstrats.

In einer bevorzugten Ausführungsform ist der wachstumsbeeinflussende Faktor ein Antibiotikum ausgewählt aus Cephalosporinen, Gyrasehemmern oder Fluorchinolonen, Makroliden, Clindamycin, Penicillinen, Sulfonamiden, Tetracyclinen, Carbapenemen und/oder Trimethoprim.

In einer weiteren Ausführungsform ist der wachstumsbeeinflussende Faktor ein Antibiotikum ausgewählt aus Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN). In einer bevorzugten Ausführungsform ist der wachstumsbeeinflussende Faktor ein Antibiotikum ausgewählt aus PIT, CTX, ERT, CAA, MEER, CIP, CAZ, AMK und/oder GEN und die Konzentration des Antibiotikums ist ausgewählt aus 0,01 µg/mL - 200 µg/mL, 0,1 µg/mL - 20 µg/mL, 0,25 µg/mL - 18 µg/mL, 0,5 µg/mL - 15 µg/mL, 1 µg/mL - 10 µg/mL, 1 µg/mL - 8 µg/mL, 1 µg/mL - 6 µg/mL oder 1 µg/mL - 4 µg/mL. In einer besonders bevorzugten Ausführungsform ist der wachstumsbeeinflussende Faktor ausgewählt als Antibiotikum PIT in einer Konzentration von 4 µg/mL, 6 µg/mL oder 8 µg/mL, weiter bevorzugt in einer Konzentration von 8 µg/mL. In einer weiteren, besonders bevorzugten Ausführungsform ist der wachstumsbeeinflussende Faktor ausgewählt als Antibiotikum CTX in einer Konzentration von 1 µg/mL, 2 µg/mL oder 4 µg/mL, weiter bevorzugt in einer Konzentration von 2 µg/mL. In einer weiteren, besonders bevorzugten Ausführungsform ist der wachstumsbeeinflussende Faktor ausgewählt als Antibiotikum ERT in einer Konzentration von 0,5 µg/mL oder 1 µg/mL, weiter bevorzugt in einer Konzentration von 0,5 µg/mL. In einer weiteren, besonders bevorzugten Ausführungsform ist der wachstumsbeeinflussende Faktor ausgewählt als Antibiotikum CAA in einer Konzentration von 4 µg/mL, 6µg/mL oder 8 µg/mL, weiter bevorzugt in einer Konzentration von 8 µg/mL. In einer weiteren, besonders bevorzugten Ausführungsform ist der wachstumsbeeinflussende Faktor ausgewählt als Antibiotikum MER in einer Konzentration von 2 µg/mL, 4µg/mL oder 8 µg/mL, weiter bevorzugt in einer Konzentration von 8 µg/mL. In einer weiteren, besonders bevorzugten Ausführungsform ist der wachstumsbeeinflussende Faktor ausgewählt als Antibiotikum CIP in einer Konzentration von 0,125 µg/mL, 0,25 µg/mL oder 0,5 µg/mL, weiter bevorzugt in einer Konzentration von 0,5 µg/mL. In einer weiteren, besonders bevorzugten Ausführungsform ist der wachstumsbeeinflussende Faktor ausgewählt als Antibiotikum CAZ in einer Konzentration von 1 µg/mL, 2 µg/mL oder 4 µg/mL, weiter bevorzugt in einer Konzentration von 4 µg/mL. In einer weiteren, besonders bevorzugten Ausführungsform ist der wachstumsbeeinflussende Faktor ausgewählt als Antibiotikum AMK in einer Konzentration von 4 µg/mL oder 8 µg/mL, weiter bevorzugt in einer Konzentration von 8 µg/mL. In einer weiteren, besonders bevorzugten Ausführungsform ist der wachstumsbeeinflussende Faktor ausgewählt als Antibiotikum GEN in einer Konzentration 1 µg/mL oder 2 µg/mL, weiter bevorzugt in einer Konzentration von 2 µg/mL.

In einer weiteren bevorzugten Ausführungsform ist das Zellsubstrat eine Mikrobe und der wachstumsbeeinflussende Faktor, ein antimikrobielles Mittel, wie ein Antibiotikum, weiter bevorzugt ist die Mikrobe ein Bakterium und das antimikrobielle Mittel ein Antibiotikum ausgewählt aus Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN), besonders bevorzugt ist die Mikrobe ein Bakterium der Familie der *Enterobacteriaceae* und das Antibiotikum ausgewählt aus PIT, CTX, ERT, CAA, MEER, CIP, CAZ, AMK und/oder GEN und die Konzentration des Antibiotikums ist ausgewählt aus 0,01 µg/mL - 200 µg/mL, 0,1 µg/mL - 20 µg/mL, 0,25 µg/mL - 18 µg/mL, 0,5 µg/mL - 15 µg/mL, 1 µg/mL - 10 µg/mL, 1 µg/mL - 8 µg/mL, 1 µg/mL - 6 µg/mL oder 1 µg/mL - 4 µg/mL, besonders bevorzugt ausgewählt aus 0,01 µg/mL - 200 µg/mL, 0,1 µg/mL - 20 µg/mL oder 0,25 µg/mL - 8 µg/mL.

In einer weiteren bevorzugten Ausführungsform ist das Zellsubstrat oder Test-Zellsubstrat eine Mikrobe und der wachstumsbeeinflussende Faktor, ein antimikrobielles Mittel, wie ein Antibiotikum oder Antimykotikum, weiter bevorzugt ist die Mikrobe ein Bakterium und das antimikrobielle Mittel ein Antibiotikum ausgewählt aus Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN), besonders bevorzugt ist die Mikrobe ein Bakterium der Familie der *Enterobacteriaceae* und das Antibiotikum ausgewählt aus PIT, CTX, ERT, CAA, MEER, CIP, CAZ, AMK und/oder GEN und die erste und zweite Aufbereitung umfasst eine Inkubation der Mikroben, wobei die Inkubation in mindestens einer Aufbereitung einen Vermehrungsschritt umfasst, am meisten bevorzugt eine Inkubation mit einer Inkubationsdauer von 4h bis 6h, wobei die Inkubation einen Vermehrungsschritt in der ersten Aufbereitung umfasst.

In einer weiteren bevorzugten Ausführungsform ist das Zellsubstrat oder Test-Zellsubstrat eine Mikrobe und der wachstumsbeeinflussende Faktor, ein antimikrobielles Mittel, wie ein Antibiotikum oder Antimykotikum, weiter bevorzugt ist die Mikrobe ein Bakterium und das antimikrobielle Mittel ein Antibiotikum ausgewählt aus Piperacillin/Tazobactam (PIT), Cefotaxim (CTX), Ertapenem (ERT), Ceftazidime/Avibactam (CAA), Meropenem (MER), Ciproflaxicin (CIP), Ceftazidim (CAZ), Amikacin (AMK) und/oder Gentamicin (GEN), besonders bevorzugt ist die Mikrobe ein Bakterium der Familie der *Enterobacteriaceae* und das Antibiotikum ausgewählt aus PIT, CTX, ERT, CAA, MEER, CIP, CAZ, AMK und/oder GEN und die Konzentration des Antibiotikums ist ausgewählt aus 0,01 µg/mL - 200 µg/mL, 0,1 µg/mL - 20 µg/mL, 0,25 µg/mL - 18 µg/mL, 0,5 µg/mL - 15 µg/mL, 1 µg/mL - 10 µg/mL, 1 µg/mL - 8 µg/mL, 1 µg/mL - 6 µg/mL oder 1 µg/mL - 4 µg/mL, besonders bevorzugt ausgewählt aus 0,01 µg/mL - 200 µg/mL, 0,1 µg/mL - 20 µg/mL oder 0,25 µg/mL - 8 µg/mL und die erste und zweite Aufbereitung umfasst eine Inkubation der Mikroben, wobei die Inkubation in mindestens einer Aufbereitung einen Vermehrungsschritt umfasst, am meisten bevorzugt eine Inkubation mit einer Inkubationsdauer von 4h bis 6h, wobei die Inkubation einen Vermehrungsschritt in der ersten Aufbereitung umfasst.

Die Begriffe "Massenspektrum" oder "massenspektrometrische Analyse" umfassen die massenspektrometrischen Rohdaten bis hin zu einer bearbeiteten Peakliste, die nur noch die Positionen und Intensitäten von Massensignalen enthält. Dabei kann ein Massenspektrum aus einer Vielzahl von Intensitätswerten in einem zusammenhängenden Messbereich als auch aus den Intensitätswerten mehrerer getrennter Massenbereiche bestehen. Das Massenspektrum kann vor der Ermittlung der Mikrobenmenge einer Signalverarbeitung unterzogen werden, die beispielsweise eine Korrektur (Subtraktion) der Basislinie, eine Glättung von Massensignalen, eine Eliminierung von Rauschsignalen und/oder eine Auswahl von Massensignalen über einem festgelegten Rauschwert umfasst. Das Massenspektrum kann ein Summenmassenspektrum sein, in dem Einzelmassenspektren addiert wurden. Bevorzugte Bereiche der ladungsbezogenen Masse (alternativ auch Masse-zu-Ladungsverhältnis genannt) liegen zwischen m/z 2000 und m/z 20000, weiter bevorzugt zwischen m/z 3000 und m/z 15000, insbesondere wenn das Test-Zellsubstrat ein Mikroorganismus ist.

Der Begriff "densitometrische Messung" oder "Densitometrie" im Sinne dieser Anmeldung umfasst alle Verfahren der direkten oder indirekten quantitativen Messung zur Bestimmung der Zellsubstratkonzentration. Die Densitometrie umfasst damit indirekte Messmethoden, wie z.B. die photometrischen Trübungsmessungen oder optischen Dichtemessung (scheinbare optische Dichte OD) auf Basis der Lichtstreuung mit Hilfe eines Photometers oder Spektrometers. Typischerweise wird die optische Dichtemessung in einem Wellenlängenbereich des Lichtspektrums von 560 bis 600 Nanometern gemessen, d.h. in einem Wellenlängenbereich, in dem keine Pigmente der Zellen absorbieren. Die OD kann als Dichte oder Extinktion für Mikroorganismen im Spektralphotometer bei 600 Nanometern oder im Filterphotometer bei 578 Nanometern gemessen werden. Weitere Methoden zur Quantifizierung der Biomasse einer Aufbereitung umfassen direkte Methoden, wie die Ermittlung des Trockengewichts, Zellproteins oder des Gesamtstickstoffs der Aufbereitung.

Die Erfindung ist vorstehend mit Bezug auf verschiedene besondere Ausführungsbeispiele beschrieben. Es versteht sich jedoch, dass diverse Gesichtspunkte oder Einzelheiten der beschriebenen Ausführungen geändert werden können, ohne vom Umfang der Erfindung abzuweichen. Weiterhin können die im Zusammenhang mit unterschiedlichen Ausführungsformen offenbarte Merkmale und Maßnahmen beliebig kombiniert werden, sofern dies einem Fachmann praktikabel erscheint. Überdies dient die vorstehende Beschreibung nur zur Veranschaulichung der Erfindung und nicht zur Einschränkung des Schutzbereichs, der ausschließlich durch die beigefügten Patentansprüche unter Berücksichtigung etwaig vorhandener Äquivalente definiert wird.

### Bevorzugte Ausführungsformen

In einer bevorzugten Ausführungsform ergibt sich ein Verfahren zum spektrometrischen Charakterisieren eines Test-Zellsubstrats, umfassend:
- Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen,
- Bereitstellen oder Erstellen erster spektrometrischer Messdaten vom Test-Zellsubstrat nach einer ersten Aufbereitung,
- Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Zellsubstrats oder davon abgeleiteten Daten aufgeführt sind,
- Bereitstellen oder Erstellen einer Teilbibliothek beinhaltend Referenzdatensätze aus der Bibliothek, für die das erste Abgleichergebnis als eine taxonomische Einordnung des Test-Zellsubstrats, insbesondere der Unterart, Art und/oder der Gattung, erlaubend bewertet wird,
- Bereitstellen oder Erstellen zweiter spektrometrischer Messdaten vom Test-Zellsubstrat nach wenigstens einer zweiten Aufbereitung unter Bedingungen, die mit den Bedingungen der ersten Aufbereitung nicht deckungsgleich sind,
- Abgleichen der zweiten spektrometrischen Messdaten oder davon abgeleiteter Daten mit der Teilbibliothek, um ein zweites Abgleichergebnis zu ermitteln, und
- Bestimmen einer Eigenschaft des Test-Zellsubstrats unter Verwendung des zweiten Abgleichergebnisses.

In Übereinstimmung mit vorherigen Ausführungsformen ergibt sich ein Verfahren, bei dem die taxonomische Einordnung eine Zuordnung eines Taxons zum Test-Zellsubstrat umfasst, das aus der Gruppe gewählt ist: Gattung (Genus), Art (Spezies), Unterart (Subspezies) und Varietät oder Serotyp.

In Übereinstimmung mit vorherigen Ausführungsformen ergibt sich ein Verfahren, bei dem das Test-Zellsubstrat Mikroorganismen, bevorzugt Bakterien, weiter bevorzugt Bakterien der Familie *Enterobacteriaceae* enthält oder daraus besteht.

In Übereinstimmung mit vorherigen Ausführungsformen ergibt sich ein Verfahren, bei dem die erste Aufbereitung und zweite Aufbereitung einen Vermehrungsschritt des Test-Zellsubstrats umfassen.

In Übereinstimmung mit vorherigen Ausführungsformen ergibt sich ein Verfahren, bei dem die zweite Aufbereitung in Gegenwart eines wachstumsbeeinflussenden Faktors ausgeführt wird.

In Übereinstimmung mit vorherigen Ausführungsformen ergibt sich ein Verfahren, bei dem die erste Aufbereitung als Wachstumskontrolle ohne Verwendung eines wachstumsbeeinflussenden Faktors ausgeführt wird.

In Übereinstimmung mit vorherigen Ausführungsformen ergibt sich ein Verfahren, bei dem ein Referenzdatensatz ein Spektrum oder ein aus einem Spektrum abgeleitetes Daten-n-tupel umfasst.

In Übereinstimmung mit vorherigen Ausführungsformen ergibt sich ein Verfahren, bei dem die zu ermittelnde Eigenschaft eine Suszeptibilität und/oder Resistenz des Test-Zellsubstrats gegenüber einem wachstumsbeeinflussenden Faktor, bevorzugt einem wachstumsbeeinflussenden Faktor, der eine negative Auswirkung auf die Vitalität und/oder Vermehrungsfähigkeit des Test-Zellsubstrat hat, weiter bevorzugt, einem wachstumsbeeinflussenden Faktor, der ein chemischer Stoff ist, der unmittelbar toxisch und/oder wachstumshemmend auf das Test-Zellsubstrat wirkt, umfasst und/oder daraus besteht.

In Übereinstimmung mit vorherigen Ausführungsformen ergibt sich ein Verfahren, bei dem die zu ermittelnde Eigenschaft eine minimale Hemmkonzentration (MHK) des wachstumsbeeinflussenden Faktors für das Test-Zellsubstrat umfasst.

In Übereinstimmung mit vorherigen Ausführungsformen ergibt sich ein Verfahren, bei dem diejenigen Referenzdatensätze, für die das erste Abgleichergebnis als keine taxonomische Einordnung des Test-Zellsubstrats erlaubend bewertet wird, beim Bereitstellen oder Erstellen der Teilbibliothek ausgeschlossen werden.

In Übereinstimmung mit vorherigen Ausführungsformen ergibt sich ein Verfahren, bei dem die Teilbibliothek beinhaltend Referenzdatensätze aus der Bibliothek, für die das erste Abgleichergebnis als eine taxonomische Einordnung des Test-Zellsubstrats, insbesondere der Art oder der Gattung, erlaubend bewertet wird, eine erste Teilbibliothek ist und bei dem eine zweite Teilbibliothek bereitgestellt oder erstellt wird, wobei die zweite Teilbibliothek diejenigen Referenzdatensätze aus der Bibliothek, für die das erste Abgleichergebnis als keine taxonomische Einordnung des Test-Zellsubstrats, insbesondere der Art oder der Gattung, erlaubend bewertet wird, umfasst, die spektrometrischen Messdaten oder davon abgeleitete Daten der zweiten Aufbereitung mit der zweiten Teilbibliothek abgeglichen werden, um ein drittes Abgleichergebnis zu ermitteln und eine Eigenschaft des Test-Zellsubstrats unter Verwendung des dritten Abgleichergebnisses bestimmt wird.

In Übereinstimmung mit vorherigen Ausführungsformen ergibt sich ein Verfahren, bei dem das Bereitstellen oder Erstellen erster und/oder zweiter spektrometrischer Messdaten vom Test-Zellsubstrat eine massenspektrometrische Analyse umfasst.

In Übereinstimmung mit vorherigen Ausführungsformen ergibt sich ein Verfahren, bei dem neben erster Aufbereitung und zweiter Aufbereitung weitere Aufbereitungen des Test-Zellsubstrats unter Bedingungen durchgeführt werden, die mit den Bedingungen der ersten Aufbereitung und zweiten Aufbereitung sowie untereinander nicht deckungsgleich sind.

In Übereinstimmung mit vorherigen Ausführungsformen ergibt sich ein Verfahren, bei dem die erste Aufbereitung und/oder zweite Aufbereitung unmittelbar auf einem Probenträger erfolgt, der als Substrat für das Bereitstellen oder Erstellen spektrometrischer Messdaten dient.

In Übereinstimmung mit vorherigen Ausführungsformen ergibt sich ein Verfahren, bei dem:
- das Test-Zellsubstrat Bakterien der Familie *Enterobacteriaceae* enthält oder daraus besteht,
- die erste Aufbereitung und zweite Aufbereitung einen Vermehrungsschritt umfassen,
- die zweite Aufbereitung in Gegenwart eines wachstumsbeeinflussenden Faktors ausgeführt wird,
- die erste Aufbereitung als Wachstumskontrolle ohne Verwendung eines wachstumsbeeinflussenden Faktors ausgeführt wird,
- die taxonomische Einordnung eine Zuordnung eines Taxons zum Test-Zellsubstrat umfasst, das aus der Gruppe gewählt ist: Gattung (Genus), Art (Spezies), Unterart (Subspezies) und Varietät oder Serotyp,
- ein Referenzdatensatz ein Spektrum oder ein aus einem Spektrum abgeleitetes Daten-n-tupel umfasst,
- die zu ermittelnde Eigenschaft eine Suszeptibilität und/oder Resistenz des Test-Zellsubstrats gegenüber einem wachstumsbeeinflussenden Faktor, ausgewählt als antimikrobielles Mittel, umfasst,
- diejenigen Referenzdatensätze, für die das erste Abgleichergebnis als keine taxonomische Einordnung des Test-Zellsubstrats erlaubend bewertet wird, beim Bereitstellen oder Erstellen der Teilbibliothek ausgeschlossen werden,
- die Teilbibliothek beinhaltend Referenzdatensätze aus der Bibliothek, für die das erste Abgleichergebnis als eine taxonomische Einordnung, insbesondere der Art oder der Gattung, des Test-Zellsubstrats erlaubend bewertet wird, eine erste Teilbibliothek ist und bei dem eine zweite Teilbibliothek bereitgestellt oder erstellt wird, wobei die zweite Teilbibliothek diejenigen Referenzdatensätze aus der Bibliothek, für die das erste Abgleichergebnis als keine taxonomische Einordnung des Test-Zellsubstrats, insbesondere der Art oder der Gattung, erlaubend bewertet wird, umfasst und die spektrometrischen Messdaten oder davon abgeleitete Daten der zweiten Aufbereitung mit der zweiten Teilbibliothek abgeglichen werden, um ein drittes Abgleichergebnis zu ermitteln,
- das dritte Abgleichergebnis als keine taxonomische Einordnung des Test-Zellsubstrats, insbesondere der Art oder der Gattung, erlaubend bewertet wird, und
- das Bereitstellen oder Erstellen erster und/oder zweiter spektrometrischer Messdaten vom Test-Zellsubstrat eine massenspektrometrische Analyse umfasst.

In einer besonders bevorzugten Ausführungsform des Verfahrens zum spektrometrischen Charakterisieren eines Test-Zellsubstrats umfasst dieses folgende Schritte oder besteht aus folgenden Schritten:
1) Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen,
2) Bereitstellen oder Erstellen erster spektrometrischer Messdaten vom Test-Zellsubstrat nach einer ersten Aufbereitung,
3) Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Zellsubstrats oder davon abgeleiteten Daten aufgeführt sind,
4) Bereitstellen oder Erstellen einer Teilbibliothek beinhaltend Referenzdatensätze aus der Bibliothek, für die das erste Abgleichergebnis als eine taxonomische Einordnung, insbesondere der Art oder der Gattung, des Test-Zellsubstrats erlaubend bewertet wird,
5) Bereitstellen oder Erstellen zweiter spektrometrischer Messdaten vom Test-Zellsubstrat nach wenigstens einer zweiten Aufbereitung unter Bedingungen, die mit den Bedingungen der ersten Aufbereitung nicht deckungsgleich sind,
6) Abgleichen der zweiten spektrometrischen Messdaten oder davon abgeleiteter Daten mit der Teilbibliothek, um ein zweites Abgleichergebnis zu ermitteln, und
7) Bestimmen einer Eigenschaft des Test-Zellsubstrats unter Verwendung des zweiten Abgleichergebnisses,
wobei die numerische Reihenfolge die Reihenfolge, in der die Verfahrensschritte ausgeführt werden, wiedergibt.

In einer besonders bevorzugten Ausführungsform des Verfahrens zum spektrometrischen Charakterisieren eines Test-Zellsubstrats umfasst dieses folgende Schritte oder besteht aus folgenden Schritten:
1) Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen,
2) Bereitstellen oder Erstellen erster spektrometrischer Messdaten vom Test-Zellsubstrat nach einer ersten Aufbereitung,
3) Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Zellsubstrats oder davon abgeleiteten Daten aufgeführt sind,
4) Bereitstellen oder Erstellen einer ersten Teilbibliothek beinhaltend Referenzdatensätze aus der Bibliothek, für die das erste Abgleichergebnis als eine taxonomische Einordnung, insbesondere der Art oder der Gattung, des Test-Zellsubstrats erlaubend bewertet wird,
5) Bereitstellen oder Erstellen einer zweiten Teilbibliothek beinhaltend Referenzdatensätze aus der Bibliothek, für die das erste Abgleichergebnis als keine taxonomische Einordnung, insbesondere der Art oder der Gattung, des Test-Zellsubstrats erlaubend bewertet wird,
6) Bereitstellen oder Erstellen zweiter spektrometrischer Messdaten vom Test-Zellsubstrat nach wenigstens einer zweiten Aufbereitung unter Bedingungen, die mit den Bedingungen der ersten Aufbereitung nicht deckungsgleich sind,
7) Abgleichen der zweiten spektrometrischen Messdaten oder davon abgeleiteter Daten mit der ersten Teilbibliothek, um ein zweites Abgleichergebnis zu ermitteln,
8) Abgleichen der zweiten spektrometrischen Messdaten oder davon abgeleiteter Daten mit der zweiten Teilbibliothek, um ein drittes Abgleichergebnis zu ermitteln, und
9) Bestimmen einer Eigenschaft des Test-Zellsubstrats unter Verwendung des dritten Abgleichergebnisses,
wobei die numerische Reihenfolge die Reihenfolge, in der die Verfahrensschritte ausgeführt werden, wiedergibt.

## Patentansprüche

1. Verfahren zum spektrometrischen Charakterisieren eines Test-Zellsubstrats, umfassend:
- Bereitstellen oder Erstellen einer Bibliothek beinhaltend eine Vielzahl Referenzdatensätze, wobei jeder Referenzdatensatz Daten enthält, die eine taxonomische Einordnung eines Zellsubstrats ermöglichen,
- Bereitstellen oder Erstellen erster spektrometrischer Messdaten vom Test-Zellsubstrat nach einer ersten Aufbereitung,
- Abgleichen der ersten spektrometrischen Messdaten oder davon abgeleiteter Daten mit der Bibliothek, um ein erstes Abgleichergebnis zu ermitteln, wobei ein Abgleichergebnis eine Liste enthält, in der Referenzdatensätze sowie deren Übereinstimmungsgrad mit spektrometrischen Messdaten eines Zellsubstrats oder davon abgeleiteten Daten aufgeführt sind,
- Bereitstellen oder Erstellen einer Teilbibliothek beinhaltend Referenzdatensätze aus der Bibliothek, für die das erste Abgleichergebnis als eine taxonomische Einordnung des Test-Zellsubstrats erlaubend bewertet wird,
- Bereitstellen oder Erstellen zweiter spektrometrischer Messdaten vom Test-Zellsubstrat nach wenigstens einer zweiten Aufbereitung unter Bedingungen, die mit den Bedingungen der ersten Aufbereitung nicht deckungsgleich sind,
- Abgleichen der zweiten spektrometrischen Messdaten oder davon abgeleiteter Daten mit der Teilbibliothek, um ein zweites Abgleichergebnis zu ermitteln, und
- Bestimmen einer Eigenschaft des Test-Zellsubstrats unter Verwendung des zweiten Abgleichergebnisses.

2. Verfahren nach Anspruch 1, bei dem die taxonomische Einordnung eine Zuordnung eines Taxons zum Test-Zellsubstrat umfasst, das aus der Gruppe gewählt ist: Gattung (Genus), Art (Spezies), Unterart (Subspezies) und Varietät oder Serotyp.

3. Verfahren nach einem der Ansprüche 1 oder 2, bei dem das Test-Zellsubstrat Mikroorganismen, bevorzugt Bakterien, weiter bevorzugt Bakterien der Familie *Enterobacteriaceae* enthält oder daraus besteht.

4. Verfahren nach einem der Ansprüche 1 oder 3, bei dem die erste Aufbereitung und zweite Aufbereitung einen Vermehrungsschritt des Test-Zellsubstrats umfassen.

5. Verfahren nach einem der Ansprüche 1 bis 4, bei dem die zweite Aufbereitung in Gegenwart eines wachstumsbeeinflussenden Faktors ausgeführt wird.

6. Verfahren nach einem der Ansprüche 1 bis 5, bei dem die erste Aufbereitung als Wachstumskontrolle ohne Verwendung eines wachstumsbeeinflussenden Faktors ausgeführt wird.

7. Verfahren nach einem der Ansprüche 1 bis 6, bei dem ein Referenzdatensatz ein Spektrum oder ein aus einem Spektrum abgeleitetes Daten-n-tupel umfasst.

8. Verfahren nach einem der Ansprüche 1 bis 7, bei dem die zu ermittelnde Eigenschaft eine Suszeptibilität und/oder Resistenz des Test-Zellsubstrats gegenüber einem wachstumsbeeinflussenden Faktor umfasst.

9. Verfahren nach Anspruch 8, bei dem die zu ermittelnde Eigenschaft eine minimale Hemmkonzentration (MHK) des wachstumsbeeinflussenden Faktors für das Test-Zellsubstrat umfasst.

10. Verfahren nach einem der Ansprüche 1 bis 9, bei dem diejenigen Referenzdatensätze, für die das erste Abgleichergebnis als keine taxonomische Einordnung des Test-Zellsubstrats erlaubend bewertet wird, beim Bereitstellen oder Erstellen der Teilbibliothek ausgeschlossen werden.

11. Verfahren nach einem der Ansprüche 1 bis 10, bei dem die Teilbibliothek beinhaltend Referenzdatensätze aus der Bibliothek, für die das erste Abgleichergebnis als eine taxonomische Einordnung des Test-Zellsubstrats erlaubend bewertet wird, eine erste Teilbibliothek ist und bei dem eine zweite Teilbibliothek bereitgestellt oder erstellt wird, wobei die zweite Teilbibliothek diejenigen Referenzdatensätze aus der Bibliothek, für die das erste Abgleichergebnis als keine taxonomische Einordnung des Test-Zellsubstrats erlaubend bewertet wird, umfasst, die spektrometrischen Messdaten oder davon abgeleitete Daten der zweiten Aufbereitung mit der zweiten Teilbibliothek abgeglichen werden, um ein drittes Abgleichergebnis zu ermitteln, und eine Eigenschaft des Test-Zellsubstrats unter Verwendung des dritten Abgleichergebnisses bestimmt wird.

12. Verfahren nach einem der Ansprüche 1 bis 11, bei dem das Bereitstellen oder Erstellen erster und/oder zweiter spektrometrischer Messdaten vom Test-Zellsubstrat eine massenspektrometrische Analyse umfasst.

13. Verfahren nach einem der Ansprüche 1 bis 12, bei dem neben erster Aufbereitung und zweiter Aufbereitung weitere Aufbereitungen des Test-Zellsubstrats unter Bedingungen durchgeführt werden, die mit den Bedingungen der ersten Aufbereitung und zweiten Aufbereitung sowie untereinander nicht deckungsgleich sind.

14. Verfahren nach einem der Ansprüche 1 bis 13, bei dem die erste Aufbereitung und/oder zweite Aufbereitung unmittelbar auf einem Probenträger erfolgt, der als Substrat für das Bereitstellen oder Erstellen spektrometrischer Messdaten dient.

15. Verfahren nach einem der Ansprüche 1 bis 14, bei dem:
- das Test-Zellsubstrat Bakterien der Familie *Enterobacteriaceae* enthält oder daraus besteht,
- die erste Aufbereitung und zweite Aufbereitung einen Vermehrungsschritt umfassen,
- die zweite Aufbereitung in Gegenwart eines wachstumsbeeinflussenden Faktors ausgeführt wird,
- die erste Aufbereitung als Wachstumskontrolle ohne Verwendung eines wachstumsbeeinflussenden Faktors ausgeführt wird,
- die taxonomische Einordnung eine Zuordnung eines Taxons zum Test-Zellsubstrat umfasst, das aus der Gruppe gewählt ist: Gattung (Genus), Art (Spezies), Unterart (Subspezies) und Varietät oder Serotyp,
- ein Referenzdatensatz ein Spektrum oder ein aus einem Spektrum abgeleitetes Daten-n-tupel umfasst,
- die zu ermittelnde Eigenschaft eine Suszeptibilität und/oder Resistenz des Test-Zellsubstrats gegenüber dem wachstumsbeeinflussenden Faktor, ausgewählt als antimikrobielles Mittel, umfasst,
- diejenigen Referenzdatensätze, für die das erste Abgleichergebnis als keine taxonomische Einordnung des Test-Zellsubstrats erlaubend bewertet wird, beim Bereitstellen oder Erstellen der Teilbibliothek ausgeschlossen werden,
- die Teilbibliothek beinhaltend Referenzdatensätze aus der Bibliothek, für die das erste Abgleichergebnis als eine taxonomische Einordnung des Test-Zellsubstrats erlaubend bewertet wird, eine erste Teilbibliothek ist und bei dem eine zweite Teilbibliothek bereitgestellt oder erstellt wird, wobei die zweite Teilbibliothek diejenigen Referenzdatensätze aus der Bibliothek, für die das erste Abgleichergebnis als keine taxonomische Einordnung des Test-Zellsubstrats erlaubend bewertet wird, umfasst und die spektrometrischen Messdaten oder davon abgeleiteten Daten der zweiten Aufbereitung mit der zweiten Teilbibliothek abgeglichen werden, um ein drittes Abgleichergebnis zu ermitteln,
- das dritte Abgleichergebnis als keine taxonomische Einordnung des Test-Zellsubstrats bewertet wird, und
- das Bereitstellen oder Erstellen erster und/oder zweiter spektrometrischer Messdaten vom Test-Zellsubstrat eine massenspektrometrische Analyse umfasst.
